# EUROPEAN PATENT APPLICATION

(11) **EP 4 484 955 A1**
(43) Date of publication of application: **01.01.2025**
(21) Application number: 23182549.8
(22) Date of filing: 29.06.2023
(51) Int. Cl.: G01N 33/569, G01N 33/68

(54) **METHOD FOR SCREENING OF EXTRACELLULAR TISSUE PEPTIDES IN MAMMALIAN TISSUE SAMPLES FOR HEALTH STATUS EVALUATION, DISEASE DIAGNOSTICS AND NEOANTIGEN DISCOVERY**

(71) Applicant: Uniwersytet Gdanski, 80-309 Gdansk (PL)
(72) Inventor: Kote, Sachin, 80-116 Gdansk (PL); Marek-Trzonkowska, Natalia, 80-752 Gdansk (PL); Pirog, Artur, 80-312 Gdansk (PL); Faktor, Jakub, 80-170 Gdansk (PL); Czaplewska, Pauina, 80-461 Gdansk (PL)
(74) Representative: Czarnik, Maciej

(57) **Abstract**

This invention refers to method for screening of extracellular tissue peptides in mammalian tissue samples for health status evaluation, disease diagnosis and neoantigen discovery. The invention involved preparing and analyzing tissue samples from solid tumors, focusing on extracellular peptidomics and tissue major histocompatibility complex (MHC) class I immunopeptidomics. The method is antibody-free, utilizing amino acid sequencing with tandem mass spectrometry. It is a straightforward, inexpensive, and rapid way to comprehensively profile the solid tumor peptidomics (extracellular peptidome and MHC class I immunopeptidomics). The method has potential to screen and used as biomarkers for health status evaluation, disease diagnostics, neoantigen discovery and prognosis of salivary gland tumors. Furthermore, the tissue MHC class I immunopeptidomics approach for neoantigen discovery, vaccine development, and design of immunotherapies.

SEQUENCE LISTING

<110> Uniwersytet Gdanski

<120> Method for screening of extracellular tissue peptides in mammalian tissue samples for health status evaluation, disease diagnostics and neoantigen discovery

<130>

<140>

<141>

<160> 77

<211> 14

<212> PRT

<213> Homo sapiens

<210> 1

<400> 1

Ser Thr Pro Leu Gly Gln Gln Gln Pro Ala Pro Arg Thr Arg

                5                   10

<211> 11

<212> PRT

<213> Homo sapiens

<210> 2

<400> 2

Ala Leu Arg Pro Ser Thr Ser Arg Ser Leu Tyr

                5                   10

<211> 13

<212> PRT

<213> Homo sapiens

<210> 3

<400> 3

Glu Thr Arg Asp Gly Gln Val Ile Asn Glu Thr Ser Gln

                5                   10

<211> 11

<212> PRT

<213> Homo sapiens

<210> 4

<400> 4

Ser Ser Leu Asn Leu Arg Glu Thr Asn Leu Asp

                5                   10

<211> 11

<212> PRT

<213> Homo sapiens

<210> 5

<400> 5

Thr Pro Leu Gly Gln Gln Gln Pro Ala Pro Arg
                5                   10

<211> 7
<212> PRT
<213> Homo sapiens
<210> 6
<400> 6

Ala Arg Met Leu Ala Gln Glu
                5

<211> 7
<212> PRT
<213> Homo sapiens
<210> 7
<400> 7

Ser Thr Ser Arg Ser Leu Tyr
                5

<211> 10
<212> PRT
<213> Homo sapiens

<210> 8

<400> 8

Leu Val Asp Thr His Ser Lys Arg Thr Leu

                5                   10

<211> 19

<212> PRT

<213> Homo sapiens

<210> 9

<400> 9

Phe Gly Gly Pro Gly Thr Ala Ser Arg Pro Ser Ser Ser Arg Ser Tyr

                5                   10                  15

Val Thr Thr

<211> 10

<212> PRT

<213> Homo sapiens

<210> 10

<400> 10

Thr Arg Asp Gly Gln Val Ile Asn Glu Thr

                5                   10

<211> 18

<212> PRT

<213> Homo sapiens

<210> 11

<400> 11

Gly Gly Pro Gly Thr Ala Ser Arg Pro Ser Ser Ser Arg Ser Tyr Val

                5                   10                  15

Thr Thr

<211> 22

<212> PRT

<213> Homo sapiens

<210> 12

<400> 12

Leu Ile Lys Thr Val Glu Thr Arg Asp Gly Gln Val Ile Asn Glu Thr

                5                   10                  15

Ser Gln His His Asp Asp

            20

<211> 14

<212> PRT

<213> Homo sapiens

<210> 13

<400> 13

Asp Ser Leu Pro Leu Val Asp Thr His Ser Lys Arg Thr Leu

                5                   10

<211> 9

<212> PRT

<213> Homo sapiens

<210> 14

<400> 14

Tyr Glu Leu Asn Pro Phe Ile Asn Arg

                5

<211> 16

<212> PRT

<213> Homo sapiens

<210> 15

<400> 15

Ser Pro Gly Gly Val Tyr Ala Thr Arg Ser Ser Ala Val Arg Leu Arg

                5                   10                  15

<211> 10

<212> PRT

<213> Homo sapiens

<210> 16

<400> 16

Asp Val Arg Gln Gln Tyr Glu Ser Val Ala

                5                   10

<211> 12

<212> PRT

<213> Homo sapiens

<210> 17

<400> 17

Ser Pro Gly Gly Val Tyr Ala Thr Arg Ser Ser Ala

                5                   10

<211> 12

<212> PRT

<213> Homo sapiens

<210> 18

<400> 18

Ser Thr Pro Leu Gly Gln Gln Gln Pro Ala Pro Arg
                5                   10
<211> 12
<212> PRT
<213> Homo sapiens
<210> 19
<400> 19
Val Glu Thr Arg Asp Gly Gln Val Ile Asn Glu Thr
                5                   10
<211> 8
<212> PRT
<213> Homo sapiens
<210> 20
<400> 20
Pro Gly Val Arg Leu Leu Gln Asp
                5
<211> 7
<212> PRT

<213> Homo sapiens

<210> 21

<400> 21

Asp Phe Ser Leu Ala Asp Ala

                5

<211> 10

<212> PRT

<213> Homo sapiens

<210> 22

<400> 22

Ser Ser Val Pro Gly Val Arg Leu Leu Gln

                5                   10

<211> 9

<212> PRT

<213> Homo sapiens

<210> 23

<400> 23

Thr Arg Asp Gly Gln Val Ile Asn Glu

                5

<211> 15

<212> PRT

<213> Homo sapiens

<210> 24

<400> 24

Gly Gly Pro Gly Thr Ala Ser Arg Pro Ser Ser Ser Arg Ser Tyr

                5                   10                  15

<211> 9

<212> PRT

<213> Homo sapiens

<210> 25

<400> 25

Ser Ser Leu Asn Leu Arg Glu Thr Asn

                5

<211> 12

<212> PRT

<213> Homo sapiens

<210> 26

<400> 26

Met His Lys Glu Glu His Glu Val Ala Val Leu Gly
                5                   10

<211> 20
<212> PRT
<213> Homo sapiens
<210> 27
<400> 27

Val Glu Thr Arg Asp Gly Gln Val Ile Asn Glu Thr Ser Gln His His
                5                   10                  15
Asp Asp Leu Glu

<211> 18
<212> PRT
<213> Homo sapiens
<210> 28
<400> 28

Gly Lys Val Gly Ala His Ala Gly Glu Tyr Gly Ala Glu Ala Leu Glu
                5                   10                  15
Arg Met

<211> 12

<212> PRT

<213> Homo sapiens

<210> 29

<400> 29

Ser Thr Arg Ser Val Ser Ser Ser Ser Tyr Arg Arg

                5                   10

<211> 11

<212> PRT

<213> Homo sapiens

<210> 30

<400> 30

Ser Ala Leu Arg Pro Ser Thr Ser Arg Ser Leu

                5                   10

<211> 8

<212> PRT

<213> Homo sapiens

<210> 31

<400> 31

Ser Leu Asn Leu Arg Glu Thr Asn
                5
<211> 8
<212> PRT
<213> Homo sapiens
<210> 32
<400> 32
Phe Thr Arg Glu Pro Gln Asp Thr
                5
<211> 7
<212> PRT
<213> Homo sapiens
<210> 33
<400> 33
Val Ser Lys Pro Asp Leu Thr
                5
<211> 12
<212> PRT
<213> Homo sapiens

<210> 34

<400> 34

Arg Gly Pro Glu Asp Glu Ile Gly Asp Pro Leu Ala

                5                   10

<211> 12

<212> PRT

<213> Homo sapiens

<210> 35

<400> 35

Ser Ser Val Pro Gly Val Arg Leu Leu Gln Asp Ser

                5                   10

<211> 12

<212> PRT

<213> Homo sapiens

<210> 36

<400> 36

Val Val Tyr Pro Trp Thr Gln Arg Phe Phe Glu Ser

                5                   10

<211> 11

<212> PRT

<213> Homo sapiens

<210> 37

<400> 37

Phe Leu Ser Phe Pro Thr Thr Lys Thr Tyr Phe

                5                   10

<211> 13

<212> PRT

<213> Homo sapiens

<210> 38

<400> 38

Asp Leu Glu Pro Thr Val Ile Asp Glu Val Arg Thr Gly

                5                   10

<211> 16

<212> PRT

<213> Homo sapiens

<210> 39

<400> 39

Tyr Gln Lys Val Val Ala Gly Val Ala Asn Ala Leu Ala His Lys Tyr

                5                   10                  15

<211> 11

<212> PRT

<213> Homo sapiens

<210> 40

<400> 40

Ser Ser Val Pro Gly Val Arg Leu Leu Gln Asp

                5                   10

<211> 12

<212> PRT

<213> Homo sapiens

<210> 41

<400> 41

Asp Asp Val Val Gly Glu Thr Val Gly Lys Thr Asp

                5                   10

<211> 12

<212> PRT

<213> Homo sapiens

<210> 42

<400> 42

Asp Ser Leu Pro Leu Val Asp Thr His Ser Lys Arg
                5                   10

<211> 16
<212> PRT
<213> Homo sapiens
<210> 43
<400> 43

Ser Pro Ala Ala Lys Pro Gly Ser Thr Pro Ser Arg Pro Ser Ser Ala
                5                   10                  15

<211> 7
<212> PRT
<213> Homo sapiens
<210> 44
<400> 44

Glu Tyr Val Gln Thr Val Lys
                5

<211> 14

<212> PRT

<213> Homo sapiens

<210> 45

<400> 45

Gln Val Ile Asn Glu Thr Ser Gln His His Asp Asp Leu Glu

                5                   10

<211> 8

<212> PRT

<213> Homo sapiens

<210> 46

<400> 46

Phe Ile Glu Asn Glu Glu Gln Glu

                5

<211> 7

<212> PRT

<213> Homo sapiens

<210> 47

<400> 47

Ala Ile Gln Phe Gly Asn Tyr

                5

<211> 10

<212> PRT

<213> Homo sapiens

<210> 48

<400> 48

Thr His Phe Asn Lys Gly Pro Ser Tyr Gly

                5                   10

<211> 10

<212> PRT

<213> Homo sapiens

<210> 49

<400> 49

Ser His Ser Leu Thr Thr Asn Ile Met Glu

                5                   10

<211> 9

<212> PRT

<213> Homo sapiens

<210> 50

<400> 50

Val Pro Val Glu Ala Val Thr Ser Lys
                5
<211> 20
<212> PRT
<213> Homo sapiens
<210> 51
<400> 51
Ala Ala Asp Pro Pro Ala Glu Asn Ser Ser Ala Pro Glu Ala Glu Gln
                5                   10                  15
Gly Gly Ala Glu
            20
<211> 26
<212> PRT
<213> Homo sapiens
<210> 52
<400> 52
Ser Glu Ser Gly Ser Phe Arg Pro Asp Ser Pro Gly Ser Gly Asn Ala
                5                   10                  15
Arg Pro Asn Asn Pro Asp Trp Gly Thr Phe

            20                  25

<211> 11

<212> PRT

<213> Homo sapiens

<210> 53

<400> 53

Lys Pro Ala Gly Pro Pro Gly Ile Leu Ala Leu

                5                   10

<211> 9

<212> PRT

<213> Homo sapiens

<210> 54

<400> 54

Tyr Glu Phe Lys Phe Pro Asn Arg Leu

                5

<211> 9

<212> PRT

<213> Homo sapiens

<210> 55

<400> 55

Asp Val Tyr Asn His Phe Leu Leu Tyr
                5
<211> 7
<212> PRT
<213> Homo sapiens
<210> 56
<400> 56
Phe Thr Leu Gln Ser Glu Leu
                5
<211> 9
<212> PRT
<213> Homo sapiens
<210> 57
<400> 57
Lys Glu Asp Asp Ala Pro Arg Thr Ile
                5
<211> 8
<212> PRT

<213> Homo sapiens

<210> 58

<400> 58

Thr Glu Phe Thr Pro Thr Glu Lys

                5

<211> 8

<212> PRT

<213> Homo sapiens

<210> 59

<400> 59

Phe Arg Ile Gly Phe Gly Ser Phe

                5

<211> 9

<212> PRT

<213> Homo sapiens

<210> 60

<400> 60

Lys Thr Asn Gly Lys Phe Leu Ile Arg

                5

<211> 11

<212> PRT

<213> Homo sapiens

<210> 61

<400> 61

Met Pro Val Gly Pro Asp Ala Ile Leu Arg Tyr

                5                   10

<211> 9

<212> PRT

<213> Homo sapiens

<210> 62

<400> 62

Gln Gly Phe Pro Asn Arg Val Val Phe

                5

<211> 9

<212> PRT

<213> Homo sapiens

<210> 63

<400> 63

Tyr Val Tyr Glu Tyr Pro Ser Arg Tyr
                5

<211> 9
<212> PRT
<213> Homo sapiens
<210> 64
<400> 64

Asn Ala Ala Glu Arg Arg Gly Pro Leu
                5

<211> 9
<212> PRT
<213> Homo sapiens
<210> 65
<400> 65

Ala Pro Arg Thr Val Ala Leu Thr Ala
                5

<211> 11
<212> PRT
<213> Homo sapiens

<210> 66

<400> 66

Met Pro Val Gly Pro Asp Ala Ile Leu Arg Tyr

                5                   10

<211> 9

<212> PRT

<213> Homo sapiens

<210> 67

<400> 67

Thr Ile Ile Asp Ile Leu Thr Lys Arg

                5

<211> 9

<212> PRT

<213> Homo sapiens

<210> 68

<400> 68

Arg Tyr Leu Glu Lys Pro Met Glu Ile

                5

<211> 9

<212> PRT

<213> Homo sapiens

<210> 69

<400> 69

Gly Thr Tyr Val Ser Ser Val Pro Arg

                5

<211> 9

<212> PRT

<213> Homo sapiens

<210> 70

<400> 70

Gly Thr Asp Pro Thr Pro Gln His Tyr

                5

<211> 10

<212> PRT

<213> Homo sapiens

<210> 71

<400> 71

Ala Ile Leu Asp Glu Thr Lys Gly Asp Tyr

                5                   10

<211> 9

<212> PRT

<213> Homo sapiens

<210> 72

<400> 72

Val Leu Lys Thr Pro Ser Ala Ala Tyr

                5

<211> 9

<212> PRT

<213> Homo sapiens

<210> 73

<400> 73

Asp Leu Val Pro Val Pro Thr Asn Leu

                5

<211> 9

<212> PRT

<213> Homo sapiens

<210> 74

<400> 74

Val Ile Glu Glu Val Pro Gly Glu Leu
                5

<211> 9
<212> PRT
<213> Homo sapiens
<210> 75
<400> 75

Tyr Gln Lys Met Tyr Gly Ile Ser Leu
                5

<211> 12
<212> PRT
<213> Homo sapiens
<210> 76
<400> 76

Lys Gly Asp Gly Pro Val Gln Gly Ile Ile Asn Phe
                5                   10

<211> 12

<212> PRT

<213> Homo sapiens

<210> 77

<400> 77

Ile Glu Val Asp Asp Glu Arg Lys Leu Arg Thr Phe

                5                   10

## Description

The invention refers to an extracellular peptidomics and major histocompatibility complex (MHC) class I immunopeptidomics sample preparation, qualitative and quantitative method from the solid tumors by antibody-free approach. The extracellular peptidomics and immunopeptidomics from solid tumors based on amino acid sequencing with tandem mass spectrometry. The invention is simple, cost-effective, fast, and comprehensive for solid tumor peptidomics profiling. Moreover, the methods enable the discovery of complex extracellular peptidomics that can be used for health status evaluation, disease diagnostics, and prognosis. The MHC class I immunopeptidomics for neoantigen discovery, vaccine development/the design of immunotherapies. Hence inventions provide valuable insights for understating the basic and applied biological questions. Neoantigen is the peptide presented by cancer-specific MHC class I (or II) molecules. The invention enable to successfully screening of MHC class I immunopeptides/peptides from the tissue. Therefore, all MHC class I peptides can be used for neoantigen discovery amongst MHC class I immunopeptides/peptides that are specific to the cancer cell and able to induce the immune response to kill the cancer cell.

Surfacome, i.e., cell surface proteins, are the vital mediator cells and their extracellular environment. Surfacome analysis from clinical samples such as tumor tissues is demanding in the scientific community. Surfacome contains complex biological information and could be the source of novel antigens so this information can be used in many different ways in the medical field. Extracellular peptidomics and immunopeptidomics are the parts of the total surfacome of patient-derived cells/tissues. Surfacome analysis could be crucial in cancer biology and immunological research because normal and cancer cells differ from their surfacome. Cancer cells have many dividing cells, variability in size and shape, small cytoplasmic volume, and losses of regular cellular surface features. Therefore, surfacome analysis (extracellular peptidomics and immunopeptidomics) provides valuable insights into cell behavior study, disease processes, biomarker discovery, and potential therapeutic targets. The concept of peptidomics has been known for at least 20-30 years. It was first applied to specific classes of bioactive peptides, such as neuropeptides. Later, with the development of analytical technology, peptides were analyzed in body fluids and various secretions. The peptidome [1] is considered as low molecular weight peptides/proteins (less than 15 kilodaltons (kDa). The tissue extracellular peptidome has a diverse range of peptides and their potential roles in various physiological and pathological processes (cell-cell communication, signaling). Tissue extracellular peptidome analysis primarily focuses on analyzing peptides present in the extracellular space. Hence these peptides indicate various biological processes, such as cell communication, disease processes, and reflect the health or disease state of the person. In addition, it provides insight into biomarker and therapeutic target discovery and drug developments, allowing the study of disease microenvironments.

Over the past decades, advanced technology allowed comprehensive peptidomics analyses. Particularly, label-free liquid chromatography with tandem mass spectrometry (LC-MS/MS) routinely identifies and quantifies thousands of peptides in a single run, providing an unprecedented opportunity to examine changes in the extracellular peptidome profile of a solid tissue or organism. Furthermore, downstream multi-bioinformatics reveal the functional understanding. Two causes primarily drove this particular direction - firstly, the expectation of biomarkers, and important peptides in reasonable concentrations, and secondly, limitations of the technology available at a certain moment. Tissue extracellular peptidomics differs from proteomics, and the requirements for successfully identifying peptides are much higher with the peptidomics approach. Expected further applications mainly concern the discovery of novel biomarker peptides, which may arise from unexpected sources, such as protein degradation, secretion, or translation of non-canonical open reading frames (ORFs).

Moreover, it is necessary to perform quantitative characterization of the whole tissue extracellular peptidome to achieve this. The traits such as the complexity of tissue peptides mixture and abundance of some classes of tissue peptides must be addressed by the analytical method intended for its analysis. Firstly, a high dynamic range must be achieved to detect and quantify peptides in a mixture containing substantial amounts. Secondly, the applied LC/MS (Liquid Chromatography with tandem mass spectrometry) method must handle peptides of different mass and hydrophobicity, providing good chromatographic separation and further MS data acquisition as possible. Currently, applied methods rely primarily on differential solubility in organic solvents or partial protein denaturation [2], [3] by heat treatment. They often suffer from the poor recovery of extracellular peptides. Here inventors show a way to largely overcome this problem, providing sample preparation without lysis and denaturation of tissue cells, guidelines enabling direct peptidomics analysis of readily available amounts of tissue material. Moreover, the tissue extracellular peptidome analysis needs to be developed more, mainly due to problems extracting low molecular weight peptides from the cell surface. Potential applications of extracellular peptidomics arise directly from the novelty of the field. The most important aspect is that it enables the discovery of novel tissue surface biomarkers of potentially exciting and essential molecules, yet similar to proteins. It allows the detection of novel drug targets such as antibody-dependent cell mediated cytotoxicity (ADCC), complement-dependent cytotoxicity (CDC), immunoconjugates, multistep targeting, among them easily-druggable and specific ones. Moreover, tissue extracellular peptides may also serve a biomarker role in health status evaluation, disease diagnostics, prognosis, or assessment of the efficiency of the treatment.

Presentation of major histocompatibility complex (MHC) ligands on the cell surface is the essential way of communicating the state of the cell to the immune system, then immune recognition and activation. Any disturbances in this process may be a potential cause of disease. For example, autoimmune disorders may emerge when the immune system incorrectly recognizes cells as non-self and attacks them. On the other hand, cancer cells may hide from immunity when no signal on the cell surface can provide an impulse for the immune system to kill the altered cells. The identification and characterization of peptides presented on the surface of cells in complex with major histocompatibility complex (MHC) molecules it also called immunopeptidomics analysis. Moreover, developing a method for detecting mutated or non-canonical peptide precursors derived from the patients' samples, such as tumor tissue, and presented by histocompatibility antigens (MHC) is highly demanded. Moreover, determining the peptide presentation pathway from the translation product to the MHC ligand in various disease scenarios will be necessary for developing vaccines, autoimmune disease therapies, cellular therapies, and anticancer and antiviral therapies. Therefore, analysis of immunopeptidomics provides valuable information about the repertoire of peptides. Furthermore, in the context of neoantigen discovery and immunotherapy, it is necessary to find the unique antigens on the cancer cell surface, also called neoantigens. The source of neoantigen is from the tumor-specific mutations or aberrantly expressed proteins, making them attractive targets for cancer immunotherapy. Therefore, neoantigen discovery is becoming a active research area and it have potential to revolutionize cancer treatment by harnessing the power of the immune system to target and eliminate tumor cells with high specificity. Immunotherapy is rapidly evolving medical field that has revolutionized the treatment of various diseases. It harnesses the power of the immune system to recognize, attack, and destroy abnormal cells, such as cancer cells or cells infected with pathogens. Immunotherapy includes immune checkpoint inhibitors, Chimeric Antigen Receptor T-cell (CAR-T) therapy, cancer vaccines, adoptive cell transfer, etc. Recently, It has shown significant advancements in the treatment of cancer, infectious diseases, autoimmune disorders, and other conditions. Ongoing research and development in immunotherapy are focused on optimizing treatment strategies, expanding the range of treatable diseases, and improving patient outcomes.

For immunopeptidomics analysis, immunoprecipitation (IP) is a widely used method to screen the [4] neoantigen/immunopeptidome. However, this approach has several technical limitations such as a) antibody dependent/biased means due to the binding specificity of the antibody, MHC subtype information is also lost in IP-based methods utilizing the pan MHC antibody [5] b) IP requires extensive washes to remove contaminants/unspecific peptides and detergent, which may lead to losses of low-affinity MHC I-associated peptides [6] [7], c) due to antibody dependence, it become very costly for higher numbers of samples preparation, d) It is time consuming and need a highly skilled personnels, e) inability to employ for non-human model due to lack of efficient antibodies for other species. The invention includes the direct isolation, qualitative, and quantitative screening of immunopeptidome on the major histocompatibility (MHC) class I molecules from the solid tumor by antibody free approach. Patient derived tissue immunopeptidome, allowing to screened neoantigens can serve as a vaccine candidate and developing T cell-based therapies for cancer. The invented approach is the comparatively quick, high throughput and cost effective pipeline, globally applicable for direct purification and quantification of neoantigens from the tumor patient samples using the unbiased mass spectrometry analytical tool.

In the context of tissue extracellular peptidomics, inventors compared scientific literature with existing tissue peptidomics and commonly used methods like proteomics, etc. In the known methods, only few reports performed qualitative or quantitative extracellular tissue peptidome analyses. Hence, inventors consider tissue peptidomes extracted from human and non human species tissues. Inventors observe a variety of attempts to perform a tissue lysis, size fractionation of polypeptides, usually by initial removal of most of the proteins by organic solvent or heat precipitation, followed by the ultracentrifugation step. Interestingly, none of those steps seem to be essential, as precipitation-only or filtering-only approaches are reported too. By looking at the amount of material used, sample preparation steps, analysis type, and analysis depth, inventors see differences in the number of identified and quantified extracellular tissue peptides between the published and current invention method. This shows no consensus or accumulated knowledge regarding the efficient and reproducible performance of separating low molecular weight patient derived extracellular tissue peptidomics. Previously described methods for tissue extracellular peptidomics analysis were characterized by low numbers of identified peptides, and few quantitative methods for peptidome analysis were introduced. There are several reasons why this approach is restricted for comprehensive analysis, and these are as follows; a) enormous complexity because patient derive tissue surface contains various elements like proteins, lipids, metabolites, signaling molecules, etc. b) high abundant proteins or lipids mask the detection of other proteins, especially low abundance proteins, and peptides, c) simultaneously limit of detection (LOD) of low abundant peptides with diagnostic potential, d) complicated and multistep sample preparation, instability of low abundant proteins or peptides (hormones or small secreted peptides), f) cell lysis, denaturation, precipitation, heat treatment methods for the removal of impurities or high abundant proteins are overwhelming that compromise the number, yield of diagnostic cellular peptides identified and crucial biological information can be lost in the background noise.

The invention of tissue extracellular peptidomics is based on sequencing natural (without modified during the sample preparation and these modifications such as denaturation of proteins, lysis, reduction, alkylation, enzymatic digestions, etc.) tissue extracellular peptides of amino acid sequences. However, there are significant differences concerning existing methods, such as in terms of sample preparation, time, and cost-wise, with the proteomics/peptidomics approach [8][9][10] for peptide biomarker findings from the clinical samples, In Table 1, inventors have compared several limitations of existing methods. These limitations include; complex or multistep sample preparation and the low number of extracellular tissue peptides identification/quantification is the significant limitations in existing methods. Moreover, invention method of is quick (need few hours for two steps sample preparation) and cheaper (do not needs any additional steps like tissue lysis, denaturation, reduction, alkylation, digestion, heating, precipitation, Tandem Mass Tag (TMT) labeling, etc.) than the existing peptidomics/proteomics method. In addition, it can be applied to mammalian tissue samples using an unbiased mass spectrometry tool. The salivary gland tumors exact causes are often unknowns and without performing thorough evaluation by medical professionals such as physical examination, imaging tests (such as an ultrasound, computerized tomography (CT) scan, or magnetic resonance imaging/MRI) the diagnosis of this kind of tumor is impossible. Therefore, the use of extracellular tissue peptidomics for early biomarkers screening and diagnosis is vital to develop an effective therapy for a salivary grand tumor and also wide range of disease models. The additional comparison of previously reported tissue peptidomics is included in **B** 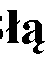 **d! Nie można odnaleźć źród** 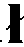 **a odwolania..**

**Table 1. Comparison of source material, sample preparation steps, and qualitative and quantitative peptide numbers between invention method (tissue extracellular peptidomics) and published methods for intracellular peptides. N/A- nonapplicable, means that the method was not developed for quantitative peptide analysis.**

| Publication | Source material | Without cell lysis and two-steps sample preparation | Cell lysis and Multi-steps sample preparation | Qualitative peptide numbers | Quantitative peptide numbers |
|---|---|---|---|---|---|
| Quantitative Peptidomics of Mouse Brain After Infection With Cyst-Forming toxoplasma gondii [11] | Whole brain | No | Yes | 2735 | 478 & 344 |
| Peptidomics Analysis Reveals Peptide PDCryab 1 Inhibits Doxorubicin-Induced Cardiotoxicity [12] | Whole mouse heart (4hearts/s ample) | No | Yes | 2945 | N/A |
| Peptidomic Analysis of Endometrial Tissue from Patients with Ovarian Endometriosis [13] | Tissue, not disclosed amount | No | Yes | 3668 | 491 |
| Peptidomic analysis on synovial tissue reveals galectin-1 derived peptide as a potential bioactive molecule against rheumatoid arthritis [14] | 5 mg tissue per sample | No | Yes | 1600 | N/A |
| Peptidome Analysis of Pancreatic Tissue Derived from T1DM Mice: Insights into the Pathogenesis and Clinical Treatments of T1DM [15] | Single mouse pancreas per sample | No | Yes | 1063 | N/A |
| Peptidome analysis of human intrauterine adhesion tissues and the identification of antifibrotic peptide [16] | 5x5x5mm tissue block per sample | No | Yes | 5191 | N/A |
| Screening and functional analysis of the differential peptides from the placenta of patients with healthy pregnancy and preeclampsia using placental peptidome [17] | Not disclosed | No | Yes | 3941 | N/A |
| Comparative peptidome profiling reveals critical roles for peptides in the pathology of pancreatic cancer [18] | Not disclosed | No | Yes | 2881 | N/A |
| Our tissue extracellular peptidome invention method | 0.3 to 0.8 gram | Yes | No | at least ≥9754 | at least ≥9329 |

The invention refers to an extracellular peptidomics and major histocompatibility complex (MHC) class I immunopeptidomics sample preparation, qualitative and quantitative method from the solid tumors by antibody-free approach. The extracellular peptidomics and immunopeptidomics from solid tumors based on amino acid sequencing with tandem mass spectrometry. The invention is simple, cost-effective, fast, and comprehensive for solid tumor peptidomics profiling. Moreover, the methods enable the discovery of complex extracellular peptidomics that can be used for biomarker discovery, health status evaluation, disease diagnosis, and prognosis. The MHC class I immunopeptidomics for neoantigen discovery, vaccine development/the design of immunotherapies. Hence inventions provide valuable insights for understating the basic and applied biological questions.

### Advantages of tissue extracellular peptidome approach

Tissue extracellular peptidome analysis is novel and tissue peptidomics is still a developing field, so inventors identify several areas that will significantly profit from the development.
1. The nature and complexity of tissue extracellular peptidome are currently not well defined. There is no reliable information on the overall abundance and cell surface secreted peptides of different disease tissue samples, especially salivary gland tumor. Experiments done with differing extraction methods can result in dramatically different qualitative and quantitative yields. To our knowledge, no experiments were performed to address this question precisely. In addition, tissue extracellular peptidome is complex and characterized by a high concentration dynamic range, similar to proteome. This will pose another analytical challenge, akin to proteomic analysis, often requiring fractionation or targeted analysis to identify and quantify (poly) peptides of interest or surfacome.
2. As mentioned, there is no consensus on the expected yield of tissue extracellular peptide isolation. This suggests that considerable sample losses are affecting reported methods. In the inventors opinion, minimum steps of sample preparation allow to comprehensive yield and biomarker discovery from tissue extracellular peptidome. The widespread belief is that cell lysis, while using only one type of denaturing agent [19] cannot provide optimal detection of peptides. Therefore, the invention does not include disrupting tissue cells by cell lysis or denaturation agents prior to any attempt at size fractionation.
3. Major difference is focusing on tissue extracellular peptidome and not proteins.
4. The invention was developed without lysis, denaturation, heating, depletion, and precipitation steps, allowing us to yield more tissue extracellular peptides and isolate peptides possibly bound to tissue/cell surface peptides.
5. The invention samples preparation is quick and more cost-effective (does not need any additional steps like heating, Tandem mass tag (TMT) labeling, etc.) than the existing peptidomics/proteomics method. In addition, it can be applied to mammalian and primary patient cells using an unbiased mass spectrometry tool.
6. The invention does not need to use additional purification or desalting kits (Ettan 2D clean) [20], [21]; all these steps are time and more cost-consuming.
7. For high sensitivity, a known method [21] used fluorescent dye (Cy3, 5) for protein labeling. Reading and imaging the proteome profile from the dye-based gel needs a specific instrument (The Typhoon instrument, GE Healthcare variable-mode imager that produces digital images of radioactive, fluorescent, or chemiluminescent samples), which again needs an investment of time and money. Therefore, altogether, it is a complex sample preparation procedure and needs an experienced person to perform these kinds of analyses.
8. The invention is completely free from additional manipulation steps such as reduction, alkylation, and enzymatic (tryptic) digestion. It also allows identifying natural, tissue extracellular peptides (which can serve as unique markers) without any modifications.
9. The method can enrich the various sizes according to the molecular weight filters used to collect tissue extracellular peptides. Thus, the filter permeability is not the limitation of the method. Invention has tested 3 kilodaltons (kDa) filter membranes, it giving significantly peptides from 0.9 to 1.8 kDa peptides enrichments.
10. The method highlights several crucial features of tissue extracellular peptidomics such as a) peptide length distribution, while using 3 kilodaltons (kDa) cutoff membrane enriches the 9-18 amino acids long peptides, b) distribution of valid value numbers on precursor level from quantified peptides, in the invention most of the peptides quantified with valid values = 5-8, which shows invention has highly confident quantification qualities tissue extracellular peptidome.
11. The method is simple and has only two steps sample preparation, a) incubation of tissue samples with an extraction buffer, allowing the dissociation of protein-peptide interactions, enrichment of tissue extracellular peptides and peptides released from complexes from tissue surface b) purification of isolated tissue extracellular peptide is performed by using hydrophilic-lipophilic balanced columns to eliminate the abundant proteins and simultaneously elute the extracellular peptides.
12. The Known method shows that they only focus on identifying proteins/peptides. However, the invention is based on performance on both qualitative and quantitative analysis. The invention is comprehensive for both qualitative and quantitative tissue extracellular peptidome analysis,
13. The instrumentation (most advanced mass spectrometry model), previously, for the separation and identification of peptides, the inventors used two-dimensional difference gel electrophoresis (2D DIGE), Surface-enhanced laser desorption/ionization (SELDI) or modified liquid chromatography-Matrix-Assisted Laser Desorption/ionization mass spectrometry (LC-MALDI), Matrix-Assisted Laser Desorption/Ionization-Time Of Flight mass spectrometry MALDI TOF/MS based platforms for various biological samples. These platforms are less sensitive and comprehensive than Exploris 480 orbitrap-based mass spectrometer. In short, the inventors have developed a novel, protein labeling by fluorescent dye, gel electrophoresis, desalting, etc., and a direct qualitative, quantitative, comprehensive tissue extracellular peptidomics approach.
14. Discovery experiments, the invention of this methodology of tissue extracellular peptidomics can be used complementary in a wide range of basic and applied research. Further development of tissue extracellular peptidomics as a method of identifying novel biologically relevant polypeptides and biomarker discovery has been applied in different disease models.

### Advantages of antibody free tissue MHC class I immunopeptidomics approach

The inventor reveals for the first time the immunopeptidome on the major histocompatibility (MHC) class I molecules from the solid tumor by antibody free approach. This is a simple, economical, and parallel approach that can be used with immunoprecipitation (IP) for neoantigen discovery. Therefore, inventors identify several areas that will significantly profit from the immunopeptidome development of solid tumor by antibody free approach.
1. The potential of an invention is to identify and quantify the MHC class I patient and disease-specific immunopeptidomes.
2. Antibody free approach is unbiased toward any antibody epitope specificity with MHC class I alleles. Therefore, the invention allowing to address the following questions; a) What is the variability and specificity of neoantigens peptidome between control and respective patients derived tissue samples, b) Neoantigen landscape specific to an individual patient, so there is a possibility of developing personalized cancer therapy.
3. Screening and discovery of common neoantigens concerning various cancer types that suites developing global immunotherapy for various kinds of cancer patients. Therefore, new customs will be developed for the entire medical field, e.g., routine screening of neoantigens.
4. The invention approach (antibody free approach) is applicable even though the starting tissue material may be limited (less than 0.5 grams). On the contrary, for immunoprecipitation (IP) base samples preparation is recommended to use at least 1 gram of tissue for obtaining optimal MHC class I peptides yield.
5. The invention approach allows for cell regeneration following β2M-dissociation, i.e., after the first time isolation of MHC class I immunopeptidome, so it can be re-analyzed after a second and subsequent perturbation. Therefore the dissociation kinetics of MHC class I immunopeptidome can be done easily.
6. The antibody free approach is simple, quick, and reproducible. Particularly the sample preparation for qualitative and quantitative tissue on the major histocompatibility (MHC) class I immunopeptidomics analysis by antibody-free approach in mammalian tissue samples. It is based on amino acid sequencing with tandem mass spectrometry with signaling intensities measurement of peptides characterized by only two steps of sample preparation as follows: a) extraction of tissue MHC class I immunopeptidomics by antibody-free approach, b) purification of tissue MHC class I immunopeptidome.
7. The invention approach (antibody free approach) is the only option for the scientific community where antibodies against MHC I molecules are not available, especially for non-human tissue samples (wild boar, cheetah, panther, etc.) to perform the immunopeptidome analysis.
8. The invention involves fewer purification steps, and no detergents are involved during the sample preparation.
9. The invention introduces no bias linked to preferential loss of low/medium/high-affinity peptides recovery.
10. Invention approach has great potential to open numerous branches for various diseases that, many based on immune response and gown in academic, medical, biotechnology, and industrial development.
11. Invention has tremendous potential to be translational, commercialization for high-throughput screening of MHC class I peptides, and subsequently, neoantigen discovery from various types of clinical tumor samples.

Therefore, the invention patient derived tissue samples and its tissue extracellular peptidomics and MHC class I immunopeptidomics data are complementary to other -omics techniques, such as proteomics. The invention is readily applicable worldwide based on clinical samples like tissue. In addition, the invention approach is comparatively faster (time-wise) and cheaper for directly finding significant biological extracellular tissue peptides biomarkers and possible neoantigens from the screened MHC class I immunopeptidomics data from the patient derived tissue samples using the unbiased mass spectrometry analytical tool.

The invention is method for direct screening and quantification of tissue extracellular peptidome and neoantigens from tissue for health status evaluation, disease diagnosis and neoantigen discovery.

This invention method can apply to all types of tissue clinical samples from human and non-human samples. The invention includes a unique sample preparation approach for qualitative and quantitative analysis of extracellular tissue and MHC class I associated immunopeptidomics by antibody-free approach. Workflow has two steps sample preparation, a. extraction b. purification of tissue peptides. Invention with few variations, particular emphasis was placed on the optimization in the first, i.e. extraction steps such as 1. prior washing steps, 2. incubation time, 3. extraction buffer pH, 4. volume of extraction buffer, provides enrichment of tissue extracellular peptidome and tissue MHC class I immunopeptidome. The second key step is purification by oasis columns (hydrophilic-lipophilic balanced) to eliminate β2 microglobulin (B2M)/abundant proteins. While simple, this method removes sufficient interference to enable tissue extracellular peptidome and tissue MHC class I immunopeptidome detection without compromising quantitative reproducibility.

### I. extraction of extracellular tissue peptidome:

Prior the extraction, the tissue samples are subjected to 2-3 washes with phosphate-buffered saline (PBS) without additives (no calcium/magnesium/phenol red) pH 7.2-7.5 and centrifuged at 500 ×g for 5 minutes at 4 °C. Then test tissue samples are incubated with an extraction buffer (citrate-phosphate buffer) with pH 3.1-3.6 prepared in liquid chromatography-mass spectrometry (LC-MS) grade ultrapure water, purity ≥ 99.9%, wherein the buffer is at a volume of 7-8 ml to 0.05-1 gram of tissue sample. The incubation step takes 2-3 minutes with mixing at a temperature of 4 to 10 °C to dissociate peptides from the highly concentrated tissue membrane surface and to collect the extracellular tissue peptidome by centrifuging at 500 ×g for 5 minutes at 4 °C. Therefore, it is characterized by the unexpected use of low pH buffer for the enrichment of tissue extracellular peptides and release from complexes from tissue surface;

### II. extraction of tissue MHC class I immunopeptidomics by antibody-free approach:

Prior the extraction, the tissue samples are subjected to 7-10 washes with phosphate-buffered saline (PBS) without additives (no calcium/magnesium/phenol red) pH 7.2-7.5 centrifuged at 500 ×g for 5 minutes at 4 °C. Then test tissue samples are incubated with a extraction buffer (citrate-phosphate buffer) with pH 3.3 prepared in liquid chromatography-mass spectrometry (LC-MS) grade ultrapure water, purity >_ 99.9%, wherein the buffer is at a volume of 5-6ml to 0.5-1 gram of tissue sample. The incubation step takes 4-5 minutes with mixing at a temperature of 4 to 10 °C to dissociate peptides MHC class I of tissue membrane surface are collected by centrifuging at 500 ×g for 5 minutes at 4 °C;

### III. Purification of extracellular tissue peptidome and MHC class I immunopeptidome,

Collected supernatants for both samples set were subjected to the Oasis cartridges (30 mg; Waters, hydrophilic-lipophilic balanced columns) to perform reverse-phase purification of polypeptides. All steps were performed at room temperature using only gravity flow, and all the detailed steps are as follows.
i) Cartridge's condition: cartridge was conditioned with 0.2% formic acid/methanol volume per volume (v/v) (in this solution final proportion of formic acid in methanol was 0.2%) twice, volume for each wash was 1ml/cartridge.
ii) Equilibration of cartridge: the cartridge was equilibrated with 0.2% formic acid/water volume per volume (v/v) (in this solution final proportion of formic acid in water was 0.2%), volume for each wash was 1ml/cartridge.
iii) Sample loading: 6-8 ml of a sample that this step was the supernatant collected after centrifugation was loaded on the cartridges (hydrophilic-lipophilic balanced columns).
iv) Washing steps: cartridge was washed three times with water containing 5% methanol/0.2% formic acid volume per volume (v/v) (in this solution final proportion of methanol and formic acid in water was 5% and 0.2%, respectively), volume for each wash was 1ml/cartridge.
v) Elution: the bound material (peptides) was eluted with 1ml water containing 80% methanol/0.2% formic acid volume per volume (v/v) (in this solution final proportion of methanol and formic acid in water was 80% and 0.2%, respectively) and then diluted to water/40% methanol/0.2% formic acid volume per volume (v/v) (in this solution final proportion of methanol and formic acid in water was 40% and 0.2%, respectively),

In the case of extracellular tissue peptidome, all filtration unit conditioning and filtration steps were performed immediately after elution. According to the instructions, to eliminate proteins equal or bigger than 15-30 kDa from peptide enriched and simultaneously elute the tissue extracellular peptides further separated with an ultrafiltration using selected molecular weight cutoff filter that has 3-30 kilo daltons (kDa), preferably 3 or 10 or 30, kDa for 120-135 minutes centrifuge at 4000 g, at 4 - 10 °C and removal of high- molecular weight proteins and other cellular compounds that have > 30 kDa; Lastly, the pipette eluted and diluted peptide solution (2ml) into the device, taking care not to touch the membrane with the pipette tip. Collect the filtrate (extracellular tissue peptidome), then evaporate it to dryness. All dried peptide samples were stored at -80 until the mass spectrometry analysis.

In the context of tissue MHC class I immunopeptidomics, to eliminate β2 microglobulin (B2M) or extracellular material equal or bigger than 3kDa from peptide enriched and further separated with ultrafiltration using selected molecular weight cutoff filter that has 3kDa molecular weight filters for 120-135 minutes centrifuge at 4000 g, at 4 - 10 °C to collect MHC class I peptides smaller than 3kDa. Lastly, the pipette eluted and diluted peptide solution (2ml) into the device, taking care not to touch the membrane with the pipette tip. Collect the filtrate (MHC class I peptides), then evaporate it to dryness. All dried peptide samples were stored at -80 until the mass spectrometry analysis.

The invention is described precisely in the example and drawing in where the following information is shown: figure 1. the workflow of screening and quantification of tissue extracellular peptidome and tissue immunopeptidome by antibody free approach is shown, figure 2. venn diagram of quantitative analysis comparing quantified of two conditions, tissue extracellular peptides in salivary gland tumor tissue (n=4) vs healthy tissue samples (n=4), figure 3A. peptide length distribution of tissue extracellular peptides identified in DIA (data-independent acquisition) spectrometry-based method in salivary gland tumor tissue and healthy tissue peptidome samples, figure 3B. distribution of valid value numbers on precursor level from quantified tissue extracellular peptides, figure 4. quality control of tissue extracellular peptidome extracts are significantly differs than MHC class I peptides, figure 5. shown the tissue extracellular peptide volcano plot shows (as dark dots) dysregulated extracellular peptides with log₂ fold change > 2 and -log p value > 2 in comparisons of salivary gland tumor tissue and healthy tissue samples, figure 6. shown STRING network analyses of identified sources of peptides with statistically different abundances in salivary gland tumor tissue and healthy tissue samples, figure 7. analysis of tissue extracellular peptide source distribution and fold change for sources of differentiating peptides, figure 8. the detailed insight into the tissue MHC class I immunopeptidome peptide identification by antibody free approach is shown, figure 9. peptide length distribution of tissue MHC class I immunopeptidome eluates from healthy and NSCLC tissue samples, figure 10. analysis of tissue MHC class I immunopeptide predictions, figure 11. gene set enrichment analysis plot of quantitatively comparing immunopeptidomes of healthy tissue to NSCLC tissue samples, figure 12. MHC class I binding evaluation of a subset of significantly changed peptides from tissue immunopeptidomics analysis of healthy tissue compared to NSCLC tissue associated with lymphocyte activation term, figure 13. volcano plot showing the dysregulated protein sources and corresponding MHC class I peptides from tissue immunopeptidomics analysis comparing healthy tissue to NSCLC tissue samples.

### Detailed description of figures:

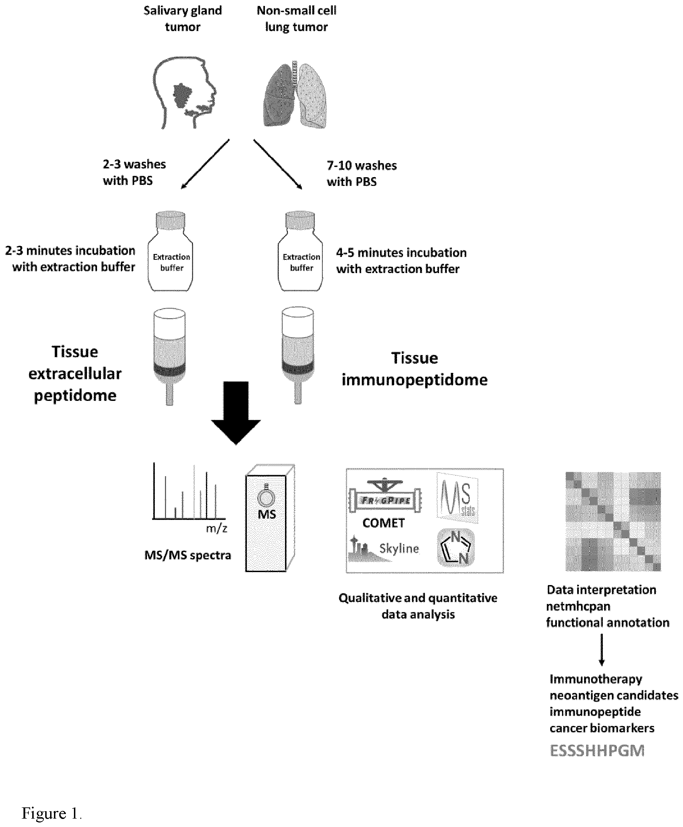

Figure 1. Workflow of screening and quantification of tissue extracellular peptides and tissue immunopeptidome by antibody free approach. The workflow needs tissue as a biological samples, and it has two steps sample preparation, a) extraction b) purification of tissue peptides. Invention with few variations in the first, i.e., extraction steps such as prior washing steps, incubation time, extraction buffer pH, provides enrichment of tissue extracellular peptidome and tissue MHC class I immunopeptidome. The second key step is purification by oasis columns (hydrophilic-lipophilic balanced) to eliminate β2 microglobulin (B2M)/abundant proteins and simultaneously elute the interest of tissue peptides. Peptides were further separated with ultrafiltration using 3 kilodaltons (3 kDa) molecular weight cutoff (MWCO) filters and for removal of high-MW proteins and other cellular compounds. While simple, this method removes sufficient interference to enable tissue extracellular peptidome and tissue MHC class I immunopeptidome detection without compromising quantitative reproducibility.

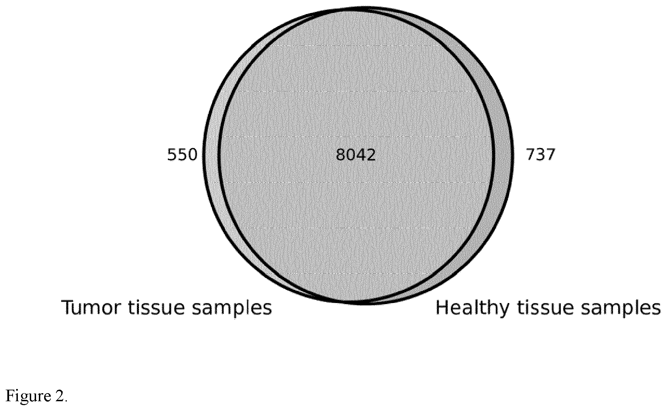

Figure 2. Venn diagram of quantitative analysis comparing quantified of two conditions, tissue extracellular peptides in salivary gland tumor tissue (n=4) vs. healthy tissue samples (n=4). The invention is comprehensively quantified at least 9329 tissue extracellular peptides.

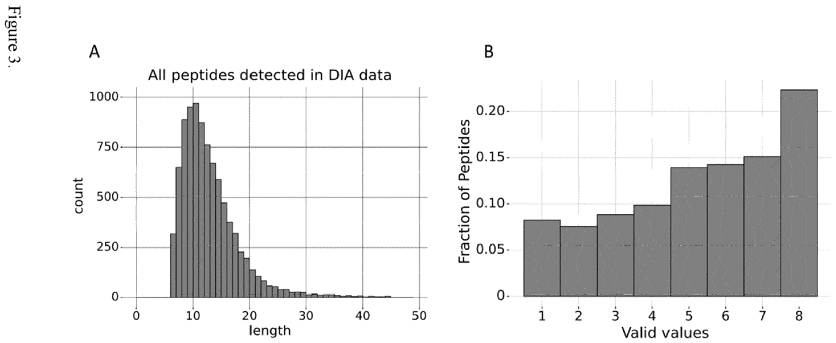

Figure 3. A. Peptide length distribution of tissue extracellular peptides identified in DIA (data-independent acquisition) spectrometry-based method in salivary gland tumor tissue and healthy tissue peptidome, samples were filtrated through a 3 kilodaltons (kDa) cutoff membrane that enriches the 8-16 amino acids long peptides. B. Distribution of valid value numbers on precursor level from quantified tissue extracellular peptides.

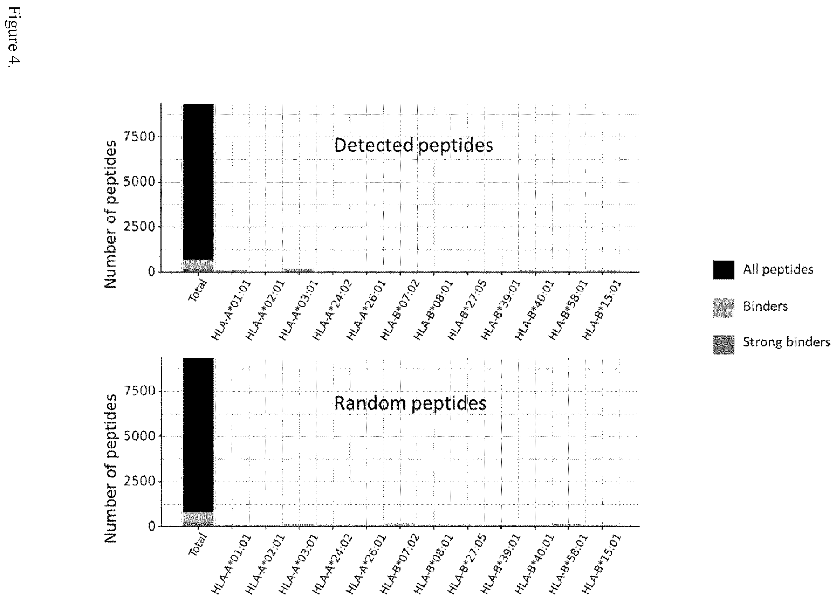

Figure 4. Quality control of tissue extracellular peptidome extracts significantly differs from MHC class I peptides. The yield of extracellular tissue peptides that differ from MHC peptides has been confirmed by comparing the detected and random/artificial peptides. The NetMHCpan binding of peptides (to any MHC molecule of known sequence) predictions revealed that extracellular tissue peptidome shows less MHC binding than random/artificial peptide set predicted MHC binders.

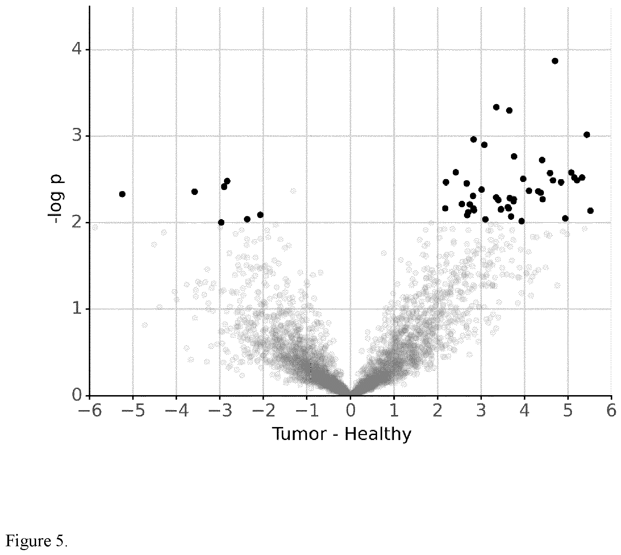

Figure 5. Tissue extracellular peptide volcano plot shows (as dark dots) dysregulated extracellular peptides with log2 fold change > 2 and -log p value > 2 in comparisons of salivary gland tumor tissue (n=4) and healthy tissue (n=4) samples.

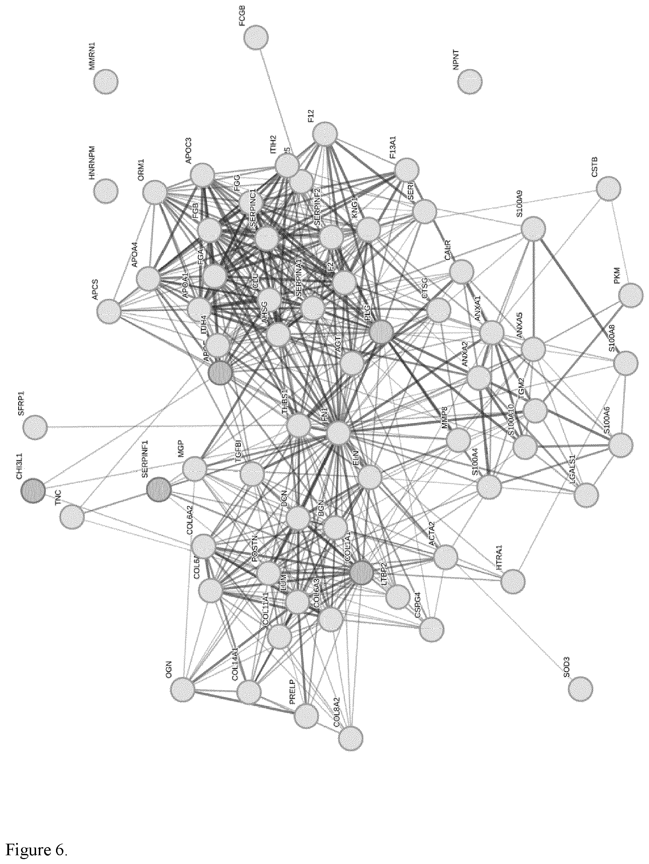

Figure 6. STRING network analyses of identified sources of peptides with statistically different abundances in salivary gland tumor tissue and healthy tissue samples. The STRING analysis of tissue extracellular peptidome composed of a majority of extracellular sources of detected peptides with high functional connectivity between obtained hits.

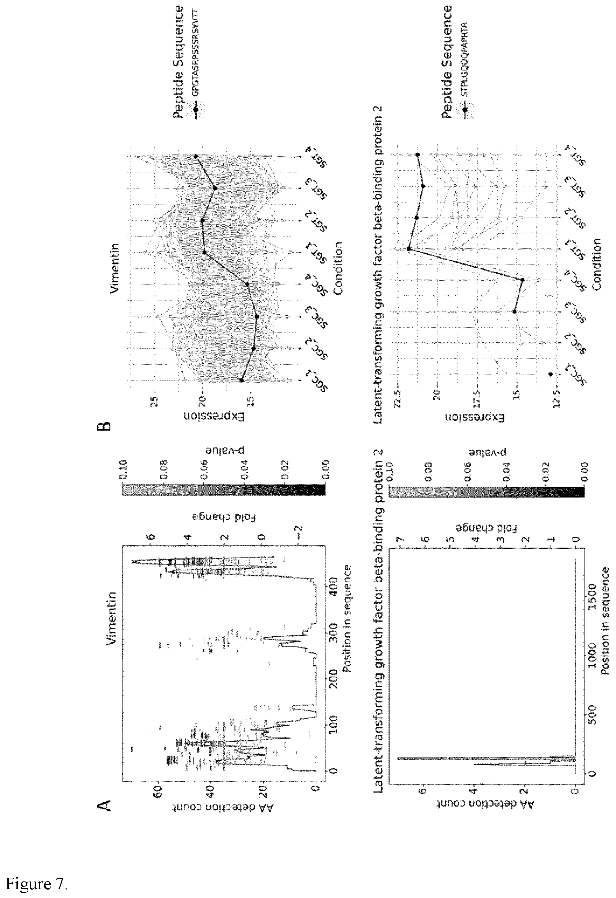

Figure 7. Analysis of tissue extracellular peptide source distribution and fold change for sources of differentiating peptides. A. amino acid location in the sequence and fold change of detected peptides in the context of their position in the full-length protein. Grayscale bar show single Welch test p-value labeling for the difference between sample groups for each peptide. B. profiles of peptide regulation (black - significantly regulated peptide, grey - other peptides potentially derived from the same protein) between comparisons of salivary gland tumor tissue and healthy tissue samples.

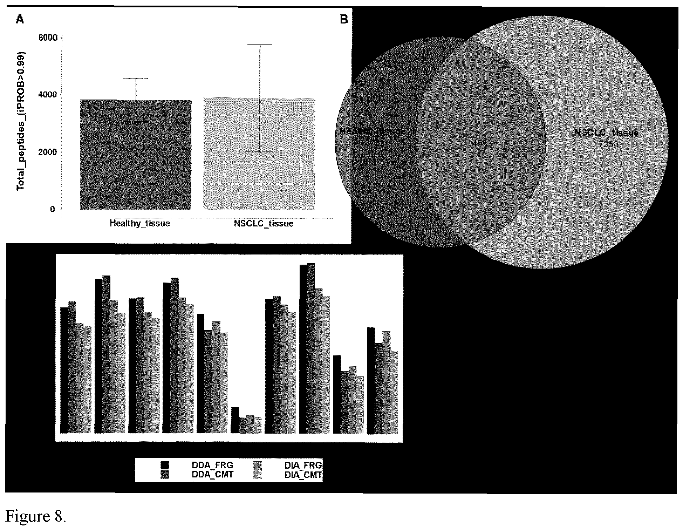

Figure 8. Detailed insight into the tissue MHC class I immunopeptidome peptide identification by antibody-free approach. A. the bar plot shows the number of tissue MHC class I immunopeptidome peptides identified (15671) in healthy tissues (n=5) and NSCLC tissues (n=5) samples; B. the venn diagram shows an overlap between peptides identified in healthy tissue and NSCLC tissue samples. The diagram highlights a considerably higher number of peptides in the NSCLC tissue group than in healthy tissue (control tissue), C. The bar plot demonstrates peptide identification per tissue using two search engines, COMET (CMT) and MS FRAGGER (FRG), and two types of mass spectrometry data acquisition, data-dependent acquisition (DDA) and data-independent acquisition (DIA). The bar plot suggests that DDA data results in more identified peptides, while the COMET and MS FRAGGER search engines perform equally effectively in peptide identification [P = patient, series A = healthy tissue, and series B = NSCLC tissue samples, 6, 8, 11, 13, and 18 numbers represent identifying different patients].

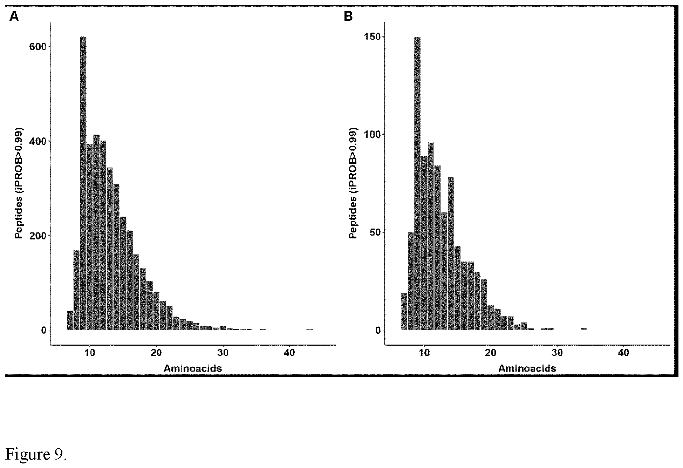

Figure 9. The representative peptide length distribution of tissue MHC class I immunopeptidome eluates from healthy (A) and NSCLC (B) tissue samples. The histograms depicting the peptide length distribution provide evidence that the majority of peptides fall within the range of 7-16 amino acid residues and higher the enrichment of 9-mer peptides, which is consistent with the characteristic length of MHC class I immunopeptidomes.

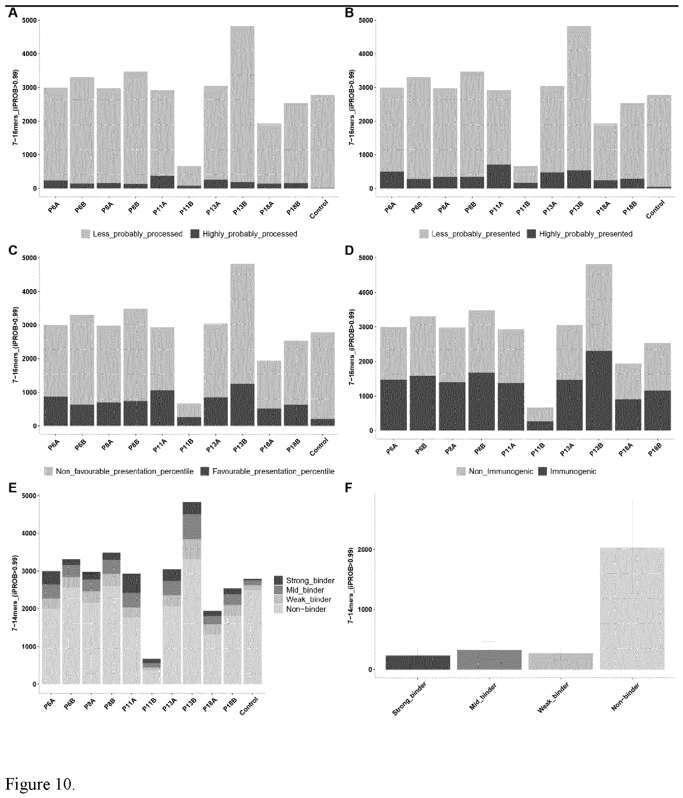

Figure 10. Analysis of tissue MHC class I immunopeptide predictions. Computational algorithms and predictive models such as the MHCflurry prediction model are used for A) antigen processing prediction, B) and C) allele-independent predictions, D) Immune Epitope Database (IEDB) MHC Class I Peptide antigenicity/immunogenicity prediction, E) and F) allele-specific predictions. By integrating these predictions, A-F inventors provided a comprehensive understanding of the MHC class I immunopeptide composition of tissue immunopeptidomics isolate by antibody free approach and further offering insights into the dynamics of immune recognition and peptide-MHC class I interactions in NSCLC tissue samples [P = patient, numbers 6, 8, 11, 13, and 18 represent identifying different patients, series A = healthy tissue, series B = NSCLC tissue samples, and control = artificial generated peptides].

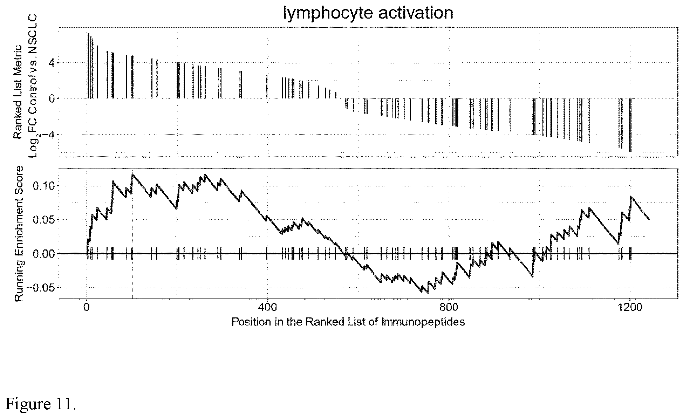

Figure 11. Gene Set Enrichment Analysis (GSEA) plot of quantitatively comparing immunopeptidomes of healthy tissue to NSCLC tissue samples. GSEA plot illustrates the enrichment of immunopeptide products of genes associated with lymphocyte activation in the analyzed dataset quantitatively comparing MHC class I immunopeptidomes of healthy tissue to NSCLC tissue samples. The enrichment pattern suggests a decreased activity of lymphocyte-related processes in NSCLC tissue expressed by an altered immunopeptidome profile.

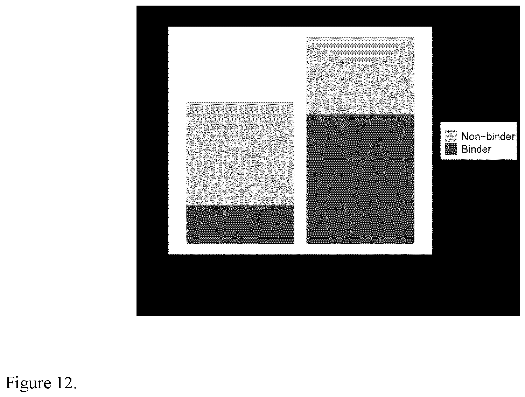

Figure 12. MHC class I binding evaluation of a subset of significantly changed peptides from tissue immunopeptidomics analysis of healthy tissue compared to NSCLC tissue associated with lymphocyte activation. The figure shows that MHC class I binders originating from lymphocyte activation are more frequently upregulated in healthy tissue than in NSCLC tissue samples.

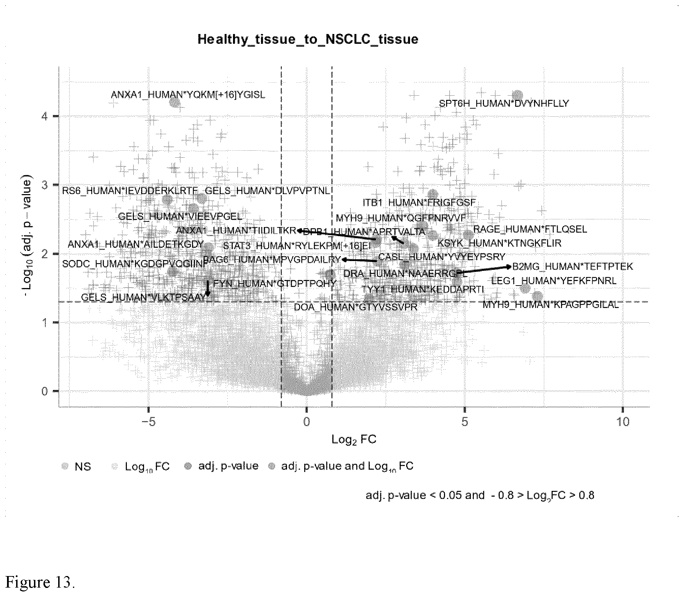

Figure 13. Volcano plot showing the dysregulated protein sources and corresponding MHC class I peptides from tissue immunopeptidomics analysis comparing healthy tissue to NSCLC tissue samples. Selected labels display the regulation of peptides that come from protein precursors linked by lymphocyte activation terms.

Detailed sample preparation and analysis methods are applied in the following examples

### Example 1

The invention method is novel, simple, and it has only two steps sample preparation and at least 4 variation steps that provides the screening and quantification of tissue extracellular peptides and tissue MHC class I immunopeptidome by antibody free approach (figure 1). Prior to starting any sample preparation steps of the tissue peptidomics (extracellular peptidomics and MHC class I immunopeptidomics) method, it is strongly recommended to prepare all the solutions using LC-MS (Liquid Chromatography with mass spectrometry) grade water, solvents, reagents, and ultraclean glass and plasticware use during methods. Moreover, all solutions and buffers should be prepared immediately before use and disposed of after one week of storage. The first step is to prepare all the necessary buffers on the same day of tissue peptidomics samples preparation.

### Buffer preparations

1^{st} buffer: citrate-phosphate buffer at pH 3.1 to 3.6 - (0.131 M citric acid/0.066 M Na2HPO4, NaCl 150 mM) and adjust the pH 3.1 to 3.6 with 1M NaOH prepared in LC-MS (Liquid Chromatography with mass spectrometry) grade water.

2^{nd} solution, 0.2% formic acid/methanol volume per volume (v/v), was prepared (the final proportion of formic acid in methanol was 0.2%).

3^{rd} solution, 0.2% formic acid/water volume per volume (v/v), was prepared (the final proportion of formic acid in water was 0.2%).

4^{th} solution wash buffer, water/5% methanol/0.2% formic acid volume per volume (v/v), was prepared (the final proportion of methanol and formic acid in water was 5% and 0.2%, respectively).

5^{th} solution elution buffer, water/80% methanol/0.2% formic acid volume per volume (v/v), was prepared (the final proportion of methanol and formic acid in water was 80% and 0.2%, respectively).

Efforts were made to develop a repeatable and effective method to obtain a sufficient amount of sample from the smallest possible amount of tissue material.

Workflow has two steps sample preparation (figure 1), a) extraction b) purification of tissue peptides. Invention with few variations in the first, i.e. extraction steps such as prior washing steps, incubation time, extraction buffer pH, and volume of extraction buffer, provides enrichment of tissue extracellular peptidome and tissue MHC class I immunopeptidome. The second key step is purification by oasis columns (hydrophilic-lipophilic balanced) to eliminate β2 microglobulin (B2M)/abundant proteins. While simple, this method removes sufficient interference to enable tissue extracellular peptidome and tissue MHC class I immunopeptidome detection without compromising quantitative reproducibility.

Invention method of sample preparation for qualitative and quantitative analysis of extracellular tissue peptidome and MHC class I immunopeptidome by antibody-free approach in mammalian tissue samples. It is based on amino acid sequencing with tandem mass spectrometry with signaling intensities measurement of peptides characterized by only two steps of sample preparation as follows:
1. extraction of extracellular tissue peptidome:
   Prior the extraction, the tissue samples are subjected to 2-3 washes with phosphate-buffered saline (PBS) without additives (no calcium/magnesium/phenol red) pH 7.2-7.5 and centrifuged at 500 ×g for 5 minutes at 4 °C. Then test tissue samples are incubated with an extraction buffer (citrate-phosphate buffer) with pH 3.1-3.6 prepared in liquid chromatography-mass spectrometry (LC-MS) grade ultrapure water, purity ≥ 99.9%, wherein the buffer is at a volume of 7-8 ml to 0.05-1 gram of tissue sample. The incubation step takes 2-3 minutes with mixing at a temperature of 4 to 10 °C to dissociate peptides from the highly concentrated tissue membrane surface and to collect the extracellular tissue peptidome by centrifuging at 500 ×g for 5 minutes at 4 °C. Therefore, it is characterized by the unexpected use of low pH buffer for the enrichment of tissue extracellular peptides and release from complexes from tissue surface;
2. extraction of tissue MHC class I immunopeptidomics by antibody-free approach:
   Prior the extraction, the tissue samples are subjected to 7-10 washes with phosphate-buffered saline (PBS) without additives (no calcium/magnesium/phenol red) pH 7.2-7.5 centrifuged at 500 ×g for 5 minutes at 4 °C. Then test tissue samples are incubated with a extraction buffer (citrate-phosphate buffer) with pH 3.3 prepared in liquid chromatography-mass spectrometry (LC-MS) grade ultrapure water, purity >_ 99.9%, wherein the buffer is at a volume of 5-6ml to 0.5-1 gram of tissue sample. The incubation step takes 4-5 minutes with mixing at a temperature of 4 to 10 °C to dissociate peptides MHC class I of tissue membrane surface are collected by centrifuging at 500 ×g for 5 minutes at 4 °C;
   Purification of extracellular tissue peptidome and MHC class I immunopeptidome, as it is shown in figure 1.

Collected supernatants for both samples set were subjected to the Oasis cartridges (30 mg; Waters, hydrophilic-lipophilic balanced columns) to perform reverse-phase purification of polypeptides. All steps were performed at room temperature using only gravity flow, and all the detailed steps are as follows.
i) Cartridge's condition: cartridge was conditioned with 0.2% formic acid/methanol volume per volume (v/v) (in this solution final proportion of formic acid in methanol was 0.2%) twice, volume for each wash was 1ml/cartridge.
ii) Equilibration of cartridge: the cartridge was equilibrated with 0.2% formic acid/water volume per volume (v/v) (in this solution final proportion of formic acid in water was 0.2%), volume for each wash was 1ml/cartridge.
iii) Sample loading: 6-8 ml of a sample that this step was the supernatant collected after centrifugation was loaded on the cartridges (hydrophilic-lipophilic balanced columns).
iv) Washing steps: cartridge was washed three times with water containing 5% methanol/0.2% formic acid volume per volume (v/v) (in this solution final proportion of methanol and formic acid in water was 5% and 0.2%, respectively), volume for each wash was 1ml/cartridge.
v) Elution: the bound material (peptides) was eluted with 1ml water containing 80% methanol/0.2% formic acid volume per volume (v/v) (in this solution final proportion of methanol and formic acid in water was 80% and 0.2%, respectively) and then diluted to water/40% methanol/0.2% formic acid volume per volume (v/v) (in this solution final proportion of methanol and formic acid in water was 40% and 0.2%, respectively),

In the case of extracellular tissue peptidome, all filtration unit conditioning and filtration steps were performed immediately after elution. According to the instructions, to eliminate proteins equal or bigger than 15-30 kDa from peptide enriched and simultaneously elute the tissue extracellular peptides further separated with an ultrafiltration using selected molecular weight cutoff filter that has 3-30 kilo daltons (kDa), preferably 3 or 10 or 30, kDa for 120-135 minutes centrifuge at 4000 g, at 4 - 10 °C and removal of high- molecular weight proteins and other cellular compounds that have > 30 kDa; Lastly, the pipette eluted and diluted peptide solution (2ml) into the device, taking care not to touch the membrane with the pipette tip. Collect the filtrate (extracellular tissue peptidome), then evaporate it to dryness. All dried peptide samples were stored at -80 until the mass spectrometry analysis.

In the context of tissue MHC class I immunopeptidomics, to eliminate β2 microglobulin (B2M) or extracellular material equal or bigger than 3kDa from peptide enriched and further separated with ultrafiltration using selected molecular weight cutoff filter that has 3kDa molecular weight filters for 120-135 minutes centrifuge at 4000 g, at 4 - 10 °C to collect MHC class I peptides smaller than 3kDa. Lastly, the pipette eluted and diluted peptide solution (2ml) into the device, taking care not to touch the membrane with the pipette tip. Collect the filtrate (MHC class I peptides), then evaporate it to dryness. All dried peptide samples were stored at -80 until the mass spectrometry analysis.

### Example 2 (Tissue extracellular peptidomics)

In the context of tissue extracellular peptidomics, the next crucial steps in the invention are mass spectrometry based measurements such as DDA (data-dependent acquisition) and DIA - (data-independent acquisition) mode. Mass spectrometry combined with chromatographic separation is necessary to identify several thousand peptides. Due to the unspecific nature of tissue extracellular peptides, successful identification requires the order of magnitude higher data quality than a standard trypsin-based proteomic sample, especially in terms of mass accuracy. Only recently, technological advances in mass spectrometry enabled the collection of data of quality acceptable for such analyses. DIA - (data-independent acquisition) method enables to the collection of quantitative data for all peptides present in the sample while maintaining good resilience to matrix interferences.

### Tissue extracellular peptidomics LC-MS/MS (Liquid Chromatography with tandem mass spectrometry) analysis

LC conditions were identical for DDA and DIA experiments. Samples were separated on the Thermo Scientific RSLC3000nano LC system, coupled to Thermo Scientific Orbitrap Exploris 480 mass spectrometer. Chromatography was performed by concentrating peptides on 0.3x5mm C18 PepMap trap column (Thermo Scientific) at the flow of 5µl/min for 10min and further separation on 0.075mm × 250mm 2µm C18 PepMap RSLC column (Thermo Scientific). The loading buffer was identical to the sample dissolution buffer, mobile phase A was 2,5% acetonitrile and 0,1% formic acid in water, and mobile phase B was 80% acetonitrile in 0.1% formic acid in water. Subsequently, peptides were separated by increasing from 2,5% mobile phase B to 35% in 80 minutes, followed increase to 60% in 15 minutes, washing with 99% B, and re-equilibration to initial conditions.

DDA data were acquired using a full scan with 120k resolution, 350-1650 Th mass range, 300% AGC (automatic gain control) target, and automatic maximum injection time. Full scans were saved as profile spectra. For fragmentation minimum intensity of 3000 was selected and dynamic exclusion was set to 20s within the +/-10ppm range from fragmented precursor ant its isotopes. The charge states 1 to 6 were allowed for fragmentation. Ion isolation window was set to 1.6 Th, resolution to 60k and normalized HCD (higher energy collisional dissociation) fragmentation energy to 30%. AGC target was set to Standard and maximum ion injection time to Auto. The fragment mass range was set to 'Define first mass' with starting m/z of 110 Th. Spectra were saved in profile mode.

DIA data were acquired using full scan with 60k resolution, 350-1450 Th mass range, 300% AGC target, and 100ms maximum injection time. DIA used 62 12 Th windows with 1 Th overlaps, covering a mass range from 350 to 1100 Th. The resolution was set o 30k, AGC to 1000%, and HCD fragmentation energy to 30%, using 3+ as the default ion charge state. Spectra were saved in profile mode.

### Tissue extracellular peptide identification

Data were analyzed mostly with the goal of quantitative characterization of intracellular peptidome. Raw data files (DDA and DIA runs) were converted to .mzml files using MSConvert with centroiding.

Converted MS data were searched using MSFragger 3.4 embedded in Fragpipe suite against the complete Homo sapiens Uniprot database (SwissProt+Trembl, all isoforms) concatenated with indexed retention time (iRT) peptide sequences and decoy reverse sequences and contaminants. The search database was constructed in Fragpipe v. 15.0 suite. Additional DDA-like data was extracted from DIA (data-independent acquisition) files by the DIA-Umpire signal extraction engine integrated with Fragpipe. The following search settings were used for MSFragger: precursor mass tolerance was set to +/-8parts per million (ppm), and fragment mass tolerance was automatically optimized. Enzyme digestion was set to non-specific and peptide length was set from 7 to 45 amino acids. The peptide mass range was set from 500 to 5000 Dalton. Variable modifications were set to: methionine oxidation, protein N-term acetylation. Data were mass recalibrated, and automatic parameter optimization setting was used to tune fragment mass tolerance. Search result filtration and validation were done automatically by Philosopher. EasyPQP were used to create the spectral libraries for further use in DIA-based quantification.

### Tissue extracellular peptide quantification

DIA data processing were performed using DIA-NN v. 1.8 All .mzml files were converted to .dia files. MBR option was selected. Precursor FDR was set to 1 %. Robust LC (high accuracy) option was selected. MS2 and MS1 mass accuracies, and scan window were automatically adjusted for each run. Retention time (RT)-dependent cross-run normalization was applied to data. Other settings in the tab were left default. Peptide quantification data were prepared by the diann-r R library. Data were preprocessed in Python to average technical replicates. Statistical analysis was performed using Perseus v.1.6.15. Peptide quantification containing more than one missing value was removed. Regulated peptides were identified using Student's test p-value cutoff of 0.01, and log2 fold change cutoff of 2.

The invention method of tissue extracellular peptidomics highlights several crucial features of extracellular peptidomics such as a) comprehensively quantified at least 9329 tissue extracellular peptides in salivary gland tumor tissue (n=4) vs healthy tissue samples (n=4) (figure 2), The venn diagram (figure 2) is to justify that it has appropriate mass extracellular tissue peptides isolation protocol and mass spectrometry-based peptidomics acquisition methods, DIA (data-independent acquisition) and DDA (data-dependent acquisition). b) peptide length distribution, while using 3 kilodaltons (kDa) cutoff membrane that enriches the 9-18 amino acids long peptides (figure 3A). Tissue extracellular peptides can be separated with ultrafiltration using a selected molecular weight cutoff filter that has 3-30 kilo daltons kDa, Thus, the filter permeability is not the limitation of the method. Figure 3B, distribution of valid value numbers on precursor level from quantified extracellular peptides. Despite high variability of the clinical samples such as tissue, 37% of the peptides were detected in at least 7 of 8 runs and therefore considered qualifying for quantification, in the invention, most of the peptides quantified with valid values between 5-8, which shows invention has highly confident quantification qualities, (figure 3B).

The next part of the invention of tissue extracellular peptidome focuses on the discovery of extracellular biomarker peptides for salivary gland tumor early diagnostics, health status evaluation, and neoantigen discovery. However, prior to do biomarker discovery, the inventors performed quality control check experiments and make sure that there is no interference of MHC class I peptidome with tissue extracellular peptidome as these both are present on the tissue/cell surface. The first quality control, the inventors check of tissue extracellular peptidome extracts, are significantly differs than MHC ligand peptides (figure 4). Tissue extracellular peptides are subjected to NetMHCpan server where predicts the binding of peptides to any MHC molecule of known sequence using artificial neural networks (ANNs). These peptides are categorized as all peptides, binders and strong binders peptides to MHC supertype representative. Figure 4, the yield of tissue extracellular peptides differ from MHC class I peptide has been confirm by comparing the detected peptides and random/artificial peptides. Tissue peptidome extract does not contain a significant amount of MHC ligand peptides. In analyzed peptide samples, only 671 out of 9321 (7.2%) peptides show potential for MHC binding, which is even less than the result for the random peptide set (799 predicted binders (8.5%). Thus, NetMHCpan binding of peptides predictions revealed that tissue extracellular peptidome shows a less percentage of MHC binding than random/artificial peptide set predicted MHC binders (figure 4). Also, length distribution presented on figure 3A do not show the typical of MHC class I peptide enrichment of 9-mer peptides only. This strongly suggests that detected peptides represent a broad population of tissue extracellular peptidome rather than a subpopulation of MHC class I peptide.

For further analysis, the invention of tissue extracellular peptidome considered only tissue extracellular peptides fully quantified in the salivary gland tumor tissue (n=4) and healthy tissue (n=4) samples. It has shown (figure 2) the identification with excellent reproducibility. Venn diagram (figure 2) comparing peptide hits between two groups salivary gland tumor tissue (n=4) and healthy tissue (n=4) samples. Statistical analysis was performed using Perseus v.1.6.15. Peptide quantification containing more than one missing value was removed. Quantification data obtained by DIA-NN were post-processed in Perseus. Significantly regulated tissue extracellular peptides were selected by using Student's test p-value cutoff of 0.01, log2 fold change cutoff of 2 and permutation-based FDR (false discovery rate) correction at 1% FDR. Results were visualized as volcano plots provided in figure 5. Tissue extracellular peptide volcano plot shows (as dark dots) dysregulated extracellular peptides with log2 fold change > 2 and -log p value > 2 in comparisons of salivary gland tumor tissue (n=4) and healthy tissue (n=4) samples. Tissue extracellular peptide quantitation experiment provides a list of peptides that significantly stratify between compared groups where quantitative analysis of the extracellular tissue peptides sequenced with a tandem mass spectrometry can be performed when signal intensity of the studied peptide is at least 4 fold higher (≥4) than the signal intensity of the matched control samples.

Salivary gland tumor extracellular tissue peptidomics pattern for health status evaluation, disease diagnostics and neoantigen discovery of Salivary gland tumor in humans is a list of at least 52 quantitative peptides. In the salivary gland tumor, extracellular peptidome higher fold change peptide sequences, and their source of the proteins identifier for each peptide is listed in Tables 2, 3, and 4, respectively. The fold change refers only to tumor extracellular quantitative peptides. It shows how many times signaling intensities in the Salivary gland tumor samples were higher than in healthy tissue samples.

**Table 2. The extracellular tissue peptide sequences and their source of the proteins identifier for each peptide is listed. Two extracellular tissue peptides are equal to or higher than 100 folds (***) in tissue salivary gland tumor patients compared to healthy tissue from the same patient.**

| Serial number | Source protein identifier | Peptide sequence | Quantitative peptides Fold change*** ≥ 100 |
|---|---|---|---|
| 1 | Latent-transforming growth factor beta-binding protein 2 | Ser-Thr-Pro-Leu-Gly-Gln-Gln-Gln-Pro-Ala-Pro-Arg-Thr-Arg | |
| 2 | Vimentin | Ala-Leu-Arg-Pro-Ser-Thr-Ser-Arg-Ser-Leu-Tyr | |

**Table 3. The extracellular tissue peptide sequences and their source of the proteins identifier for each peptide is listed. Fourteen extracellular tissue peptides are equal to or higher than 20 folds (**) in tissue salivary gland tumor patients compared to healthy tissue from the same patient.**

| Serial number | Source protein identifier | Peptide sequence | Quantitative peptides Fold change** ≥ 20 |
|---|---|---|---|
| 3 | Vimentin | Glu-Thr-Arg-Asp-Gly-Gln-Val-Ile-Asn-Glu-Thr-Ser-Gln | |
| 4 | Vimentin | Ser-Ser-Leu-Asn-Leu-Arg-Glu-Thr-Asn-Leu-Asp | |
| 5 | Latent-transforming growth factor beta-binding protein 2 | Thr-Pro-Leu-Gly-Gln-Gln-Gln-Pro-Ala-Pro-Arg | |
| 6 | Calmodulin-like protein 5 | Ala-Arg-Met-Leu-Ala-Gln-Glu | |
| 7 | Vimentin | Ser-Thr-Ser-Arg-Ser-Leu-Tyr | |
| 8 | Vimentin | Leu-Val-Asp-Thr-His-Ser-Lys-Arg-Thr-Leu | |
| 9 | Vimentin | Phe-Gly-Gly-Pro-Gly-Thr-Ala-Ser-Arg-Pro-Ser-Ser-Ser-Arg-Ser-Tyr-Val-Thr-Thr | |
| 10 | Vimentin | Thr-Arg-Asp-Gly-Gln-Val-Ile-Asn-Glu-Thr | |
| 11 | Vimentin | Gly-Gly-Pro-Gly-Thr-Ala-Ser-Arg-Pro-Ser-Ser-Ser-Arg-Ser-Tyr-Val-Thr-Thr | |
| 12 | Vimentin | Leu-Ile-Lys-Thr-Val-Glu-Thr-Arg-Asp-Gly-Gln-Val-Ile-Asn-Glu-Thr-Ser-Gln-His-His-Asp-Asp | |
| 13 | Vimentin | Asp-Ser-Leu-Pro-Leu-Val-Asp-Thr-His-Ser-Lys-Arg-Thr-Leu | |
| 14 | Matrix Gla protein | Tyr-Glu-Leu-Asn-Pro-Phe-Ile-Asn-Arg | |
| 15 | Vimentin | Ser-Pro-Gly-Gly-Val-Tyr-Ala-Thr-Arg-Ser-Ser-Ala-Val-Arg-Leu-Arg | |
| 16 | Vimentin | Asp-Val-Arg-Gln-Gln-Tyr-Glu-Ser-Val-Ala | |

**Table 4. The extracellular tissue peptide sequences and their source of the proteins identifier for each peptide is listed. Thirty-six extracellular tissue peptides are equal to or higher than 4 folds (*) in tissue salivary gland tumor patients compared to healthy tissue from the same patient.**

| Serial number | Source protein identifier | Peptide sequence | Quantitative peptides Fold change* ≥ 4 |
|---|---|---|---|
| 17 | Vimentin | Ser-Pro-Gly-Gly-Val-Tyr-Ala-Thr-Arg-Ser-Ser-Ala | |
| 18 | Latent-transforming growth factor beta-binding protein 2 | Ser-Thr-Pro-Leu-Gly-Gln-Gln-Gln-Pro-Ala-Pro-Arg | |
| 19 | Vimentin | Val-Glu-Thr-Arg-Asp-Gly-Gln-Val-Ile-Asn-Glu-Thr | |
| 20 | Vimentin | Pro-Gly-Val-Arg-Leu-Leu-Gln-Asp | |
| 21 | Vimentin | Asp-Phe-Ser-Leu-Ala-Asp-Ala | |
| 22 | Vimentin | Ser-Ser-Val-Pro-Gly-Val-Arg-Leu-Leu-Gln | |
| 23 | Vimentin | Thr-Arg-Asp-Gly-Gln-Val-Ile-Asn-Glu | |
| 24 | Vimentin | Gly-Gly-Pro-Gly-Thr-Ala-Ser-Arg-Pro-Ser-Ser-Ser-Arg-Ser-Tyr | |
| 25 | Vimentin | Ser-Ser-Leu-Asn-Leu-Arg-Glu-Thr-Asn | |
| 26 | Interferon-induced transmembrane protein 1 | Met-His-Lys-Glu-Glu-His-Glu-Val-Ala-Val-Leu-Gly | |
| 27 | Vimentin | Val-Glu-Thr-Arg-Asp-Gly-Gln-Val-Ile-Asn-Glu-Thr-Ser-Gln-His-His-Asp-Asp-Leu-Glu | |
| 28 | Hemoglobin subunit alpha | Gly-Lys-Val-Gly-Ala-His-Ala-Gly-Glu-Tyr-Gly-Ala-Glu-Ala-Leu-Glu-Arg-Met | |
| 29 | Vimentin | Ser-Thr-Arg-Ser-Val-Ser-Ser-Ser-Ser-Tyr-Arg-Arg | |
| 30 | Vimentin | Ser-Ala-Leu-Arg-Pro-Ser-Thr-Ser-Arg-Ser-Leu | |
| 31 | Vimentin | Ser-Leu-Asn-Leu-Arg-Glu-Thr-Asn | |
| 32 | Clusterin | Phe-Thr-Arg-Glu-Pro-Gln-Asp-Thr | |
| 33 | Vimentin | Val-Ser-Lys-Pro-Asp-Leu-Thr | |
| 34 | Prolow-density lipoprotein receptor-related protein 1 | Arg-Gly-Pro-Glu-Asp-Glu-Ile-Gly-Asp-Pro-Leu-Ala | |
| 35 | Vimentin | Ser-Ser-Val-Pro-Gly-Val-Arg-Leu-Leu-Gln-Asp-Ser | |
| 36 | Hemoglobin subunit delta | Val-Val-Tyr-Pro-Trp-Thr-Gln-Arg-Phe-Phe-Glu-Ser | |
| 37 | Hemoglobin subunit alpha | Phe-Leu-Ser-Phe-Pro-Thr-Thr-Lys-Thr-Tyr-Phe | |
| 38 | Tubulin alpha-1B chain | Asp-Leu-Glu-Pro-Thr-Val-Ile-Asp-Glu-Val-Arg-Thr-Gly | |
| 39 | Hemoglobin subunit delta | Tyr-Gln-Lys-Val-Val-Ala-Gly-Val-Ala-Asn-Ala-Leu-Ala-His-Lys-Tyr | |
| 40 | Vimentin | Ser-Ser-Val-Pro-Gly-Val-Arg-Leu-Leu-Gln-Asp | |
| 41 | Microtubule-associated protein 4 | Asp-Asp-Val-Val-Gly-Glu-Thr-Val-Gly-Lys-Thr-Asp | |
| 42 | Vimentin | Asp-Ser-Leu-Pro-Leu-Val-Asp-Thr-His-Ser-Lys-Arg | |
| 43 | Microtubule-associated protein RP/EB family member 2 | Ser-Pro-Ala-Ala-Lys-Pro-Gly-Ser-Thr-Pro-Ser-Arg-Pro-Ser-Ser-Ala | |
| 44 | Annexin A1 | Glu-Tyr-Val-Gln-Thr-Val-Lys | |
| 45 | Vimentin | Gln-Val-Ile-Asn-Glu-Thr-Ser-Gln-His-His-Asp-Asp-Leu-Glu | |
| 46 | Annexin A1 | Phe-Ile-Glu-Asn-Glu-Glu-Gln-Glu | |
| 47 | Biglycan | Ala-Ile-Gln-Phe-Gly-Asn-Tyr | |
| 48 | Calponin-3 | Thr-His-Phe-Asn-Lys-Gly-Pro-Ser-Tyr-Gly | |
| 49 | Fibrinogen alpha chain | Ser-His-Ser-Leu-Thr-Thr-Asn-Ile-Met-Glu | |
| 50 | Src substrate cortactin | Val-Pro-Val-Glu-Ala-Val-Thr-Ser-Lys | |
| 51 | Y-box-binding protein 1 | Ala-Ala-Asp-Pro-Pro-Ala-Glu-Asn-Ser-Ser-Ala-Pro-Glu-Ala-Glu-Gln-Gly-Gly-Ala-Glu | |
| 52 | Fibrinogen alpha chain | Ser-Glu-Ser-Gly-Ser-Phe-Arg-Pro-Asp-Ser-Pro-Gly-Ser-Gly-Asn-Ala-Arg-Pro-Asn-Asn-Pro-Asp-Trp-Gly-Thr-Phe | |

To further understand biological significance inferred from tissue extracellular peptidome quantitative data in salivary gland tumor patients, employed Search Tool for the Retrieval of Interacting Genes/Proteins (STRING) was carried out (figure 6). Initially, generated interactome maps in Search Tool for the Retrieval of Interacting Genes/Proteins (STRING) to determine characteristic nodes enriched in tissue salivary gland tumor patients as compared with healthy tissue from the same patient. STRING aims to discriminate specifically between tissue tumor and healthy samples. String analyses of significantly dysregulated protein identifiers in extracellular matrix proteins or collagen-containing extracellular matrix. The filtered subset of cellular components terms describing extracellular matrix unique protein identifiers corresponding to significantly dysregulated peptides (Student's test p-value ≤ 0.01 and fold-change ≥ 2) resulting from a comparison of salivary gland tissue tumor and healthy samples. Analysis shows interesting STRING nodes of identified sources of peptides with statistically different abundances in salivary gland tumor tissue and healthy tissue samples. The STRING analysis of tissue extracellular peptidome composed of a majority of extracellular sources of detected peptides with high functional connectivity between obtained hits (figure 6). Thus, interaction networks provide another potent way of determining key enriched protein nodes from which a predictive role of the tissue extracellular peptide could be determined.

The tissue extracellular peptidomics approach allows us to screen the peptide level expression in different tissue conditions. The screening facilitates understanding the exact biological mechanism and the discovery of tissue extracellular peptide biomarkers for various disease conditions. Therefore, the final analysis of tissue extracellular peptidomics shows that differential abundance profile of multiple peptides between the salivary gland tumor patients compared with healthy tissue samples. Invention quantitative data shows even single proteins are the source of various tissue extracellular peptides that may have differential abundance profiles. The quantitative analysis of the intracellular peptides sequenced with tandem mass spectrometry can be performed when signal intensity of the studied peptide is at t-test and permutation-based false discovery rate (FDR) correction at 1% FDR (false discovery rate) between salivary gland tumor with healthy tissue samples. Inventors are able to quantify these fascinating examples, namely Vimentin and Latent-transforming growth factor beta-binding protein 2 (LTBP2) proteins are sources of subsets of differentially abundant peptides detected in salivary gland tumor tissue compared with healthy tissue samples experiment (figure 7). Analysis of tissue extracellular peptide source distribution and fold change for sources of differentiating peptides, especially amino acid location in the sequence and fold change of detected peptides in the context of their position in full-length protein (figure 7). Moreover, the difference between sample groups for each peptide and profiles of peptide regulation between comparisons of salivary gland tumor tissue and healthy tissue samples (figure 7).

Inventors considered the quantitate analysis and the following two peptides as an example to show that the tissue extracellular peptidomics has potential applications towards the tissue derived biomarker discovery. Analysis data revealed two peptides, one derived from Vimentin - an intermediate filament protein typical for mesenchymal cells, and second derived from Latent-transforming growth factor beta-binding protein 2 (LTBP2) - an extracellular matrix protein and growth factor binding protein. Vimentin peptide comes from the N-terminal part of the protein (17 to 34 AA) and is upregulated in salivary gland tumor tissue samples, however after inspecting the data, inventors have found that almost all Vimentin-derived peptides show a similar trend (Figure 7). These peptides are distributed non-randomly on the protein sequence, concentrating in the N-terminal (1-140 AA), C-terminal (410-466 AA), and region in the middle of the sequence (260-320 AA). These peptides may simply represent the pattern of protein degradation, signaling modulation. In the case of LTBP2, the peptide in question was heavily upregulated in salivary gland tumor tissue samples. Inventors were also able to observe that other peptides coming from this protein show identical trend. All of them were derived from a relatively well-defined region located near N-terminus, roughly from 70 to 140 AA. Therefore, the vimentin and LTBP2 derived overexpressed in salivary gland tumor tissue samples indicating to play a role in salivary gland tumor progression and are the potential biomarkers for health status evaluation, disease diagnostics and neoantigen discovery. In addition, the tissue extracellular peptidomics approach clearly discriminates the peptides which are differentially regulated and non-regulated peptides even though the source of the protein is the same (figure 7). It suggests that there are differential isoform degradation, partial degradation or other more complex process concerning these proteins in this cellular model. Therefore, these correlations between different peptides with potentially identical sources can be helpful for the biological interpretation of the results for salivary gland tumor samples. Hence the novel approach for tissue extracellular peptidome sample preparation is comprehensive, qualitative, and quantitative and provides insight into biomarker discovery. Therefore, it will revolutionize the health status evaluation, disease diagnostics and neoantigen discovery field and show a roadmap to the Point of Care Diagnostic (POCD) developments. Ultimately, it will improve the quality of life and be applied to the globe. Therefore, the invention will bring breakthroughs in Point of Care Diagnostic (POCD) platforms and revolutionize the medical field. It is ready to be employed for all kinds (human/non-human) of control/disease serum samples in the future.

### Example 3 (Tissue MHC class I immunopeptidome by antibody free approach)

Tissue MHC class I immunopeptidome by antibody free approach is simple, economical and parallel approach that can be used with immunoprecipitation (IP) for neoantigen discovery. After the isolation and purification of tissue based MHC class I immunopeptidome by antibody free approach, the next crucial step in the invention is Mass spectrometry-based measurements such as DDA (data-dependent acquisition) and DIA - (data-independent acquisition) mode. Mass spectrometry combined with chromatographic separation is necessary to identify several thousand peptides. Due to the unspecific nature of MHC class I immunopeptidome, successful identification requires the order of magnitude higher data quality than a standard trypsin-based proteomic sample, especially in terms of mass accuracy. Only recently, technological advances in mass spectrometry enabled the collection of data of quality acceptable for such analyses. DIA - (data-independent acquisition) method enables to a collection of quantitative data for all peptides present in the sample while maintaining good resilience to matrix interferences.

### MHC class I immunopeptidome LC-MS/MS (Liquid Chromatography with tandem mass spectrometry) analysis

All dried immunopeptide samples were resuspended in 30 µl loading buffer (0.08% trifluoroacetic acid (TFA) in water with 2.5% acetonitrile (ACN). Each sample was spiked with retention time standard iRT peptides (Biognosys, Switzerland) according to the manufacturer's guidelines. Following, 6 µl of the dissolved sample were injected into a Thermo Scientific, UltiMate^{™} 3000 RSLCnano System (Thermo Scientific, MA, USA) liquid chromatography. Peptides were loaded on an Acclaim^{™} PepMap^{™} 100 C18, 5µm, ID= 1 mm, Length = 5 mm, (Thermo Scientific, MA, USA) trap column using a 5 µl/min loading buffer flow. Subsequently, peptides were separated on PepMap^{™} 100 C18, 5µm, ID= 1 mm, Length = 5 mm, particle size = 2µm (cat. no: 164534) (Thermo Scientific, MA, USA) analytical column. Analytical separation was performed by a non-linear increase of mobile phase B (0.1% formic acid (FA) in ACN (v/v)) in a mobile phase A (0.1% FA in water (v/v)). The non-linear gradient started at 2.5% B, linearly increasing up to 35% B in 180 min, followed by a linear increase up to 60% B in the next 15 min with a constant flow rate of 300 nl/min. Immunopeptides eluting from the column were ionized in a nano-electrospray ion source (NSI) and were introduced to Orbitrap Exploris^{™} 480 Mass Spectrometer (Thermo Scientific, MA, USA) online coupled to the liquid chromatograph.

The data-dependent acquisition (DDA) method, briefly, Exploris 480 acquired the data in data-dependent peptide mode. The full scan was operated in profile mode with 120000 resolution, and the precursor MS scan range was set from m/z 350 Th to m/z 1650 Th. Normalized AGC target was set to 300% with 100 msec maximum injection time. Each MS scan was followed by a fragmentation of the top 20 the most intense precursor ions and the acquisition of their MS/MS spectra. The dynamic mass exclusion was set to 20 see after the first precursor ion fragmentation. Precursor isotopologues were excluded, and precursor isolation mass tolerance was set to 10 ppm. The minimum precursor ion intensity was set to 3.0e3, and only precursor charge states of +1 to +5 were included in the experiment. The precursor isolation window was set to 1.6 Th. Normalized collision energy type with fixed collision energy mode was selected. The collision energy was set to 30%. Orbitrap resolution was set to 60000. The normalized AGC target was set to 100% with an automatic setting on maximum injection time, and data type was centroid.

The data independent acquisition (DIA) method was used to retrieve quantitative tissue immunopeptidomics data. LC separation parameters were kept identical to DDA acquisition. Orbitrap Exploris 480 mass spectrometer operated in positive polarity data-independent mode (DIA) mode accompanied by a full-scan with 60000 resolution. The full-scan mass range was set from m/z 350 Th up to m/z 1450 Th, and the normalized AGC target was set to 300% with 100 msec maximum injection time. The DIA method cycled in a mass range from m/z 350 Th up to m/z 1100 Th with 12 Th window width and 1 Th overlap. The method performed 62 precursor windows/scan events per one cycle. Normalized collision energy type with fixed collision energy mode was selected. The collision energy was set to 30% and orbitrap resolution to 30000. Normalized AGC target was set to 1000% with the automatic setting of maximum injection time. Data type was profile.

### Data analysis of MHC class I immunopeptidome

Qualitative tissue MHC class I immunopeptidome eluate analysis and spectral library generation; a multi-search engine strategy has been employed to identify immunopeptides in both DDA and DIA MS data. Raw DDA files were centroided and converted into mzML and mzXML format using MSconvert version: 3.0.19094. DIA raw files were directly processed in DIA Umpire SE embedded in FragPipe (v.15). Extracted pseudo-DDA data were converted to centroided mzML and mzXML format. mzML files were searched using MSFragger 3.4 and mzXML files using Comet (Release 2020.01, revision 2) engines against Homo sapiens SwissProt+UniProt and iRT (Biognosys, Switzerland) search database (12_2022). The target search database was concatenated with a reverse decoy database containing reversed target sequences of targets and common contaminant protein sequences. The following search settings were used for MSFragger: precursor mass tolerance was set to +-8 ppm and fragment mass tolerance to 10 ppm. Enzyme digestion was set to non-specific and peptide length was set from 7 to 45 amino acids. Peptide mass range was set from 200 to 5000 Da. Variable modifications were set to: methionine oxidation and protein N-term acetylation. Data were mass recalibrated, and automatic parameter optimization setting was used to tune fragment mass tolerance. The output file format was set to pep.XML. Comet precursor mass tolerance was set to 8 ppm and fragment mass tolerance to 8 ppm. The rest of the settings were identical to MSFragger. The search results (pep.XML) files were processed with PeptideProphet and iProphet as part of TPP 5.2.0. The peptide identifications in retrieved pep.XML files were further processed in R for visualization and visualized in ggplot2 and eulerr 6.1.1. R packages. The pep.XML files with recalculated peptide iprobabilities were further processed in Skyline-daily (64-bit, 20.1.9.234) to a spectral library. A blank document in Skyline-daily was set prior to loading pep.XML files. In "Peptide settings," a "Library" tab was selected. An option of "Build" was selected and in the newly popped-up window the "Cut-off score" was set to 0.99 and as an iRT standard peptides were selected "Biognosys-11 (iRT-C18)". All pep.XML files were loaded. Next, in the "Digestion" tab in the "Background proteome" section the "add" option was selected. In the newly popped up window the background proteome was created from the .FASTA file that was previously used as a search library. Next, in the "Library" tab, "Libraries" section the newly built spectral library was selected and the "Explore" button was clicked to open the library. In the library window an "Associate proteins" option was checked and the "Add all" button was clicked. The retention time calculator was automatically generated based on the iRT peptide retention time values observed in library pep.XML files. Such a prepared Skyline file was saved on the PC hard disc drive.

### Data-Independent Acquisition (DIA) data extraction

DIA data extraction was performed using Skyline-daily (64-bit, 20.1.9.234) with spectral library and document generated in the previous step. In the "Peptide settings" tab, the "Max. missed cleavages" was set to 0. Next, in the "Filter" tab, a maximum peptide length was set from 4 to 200 amino acids. The "Exclude N terminal AAs" option was set to zero. A function of the "Auto-select all matching peptides" was selected. In the "Modifications" tab, no modifications were selected. In the "Library" tab, in the "Libraries" window, a spectral library that was created in the previous step was selected. Other settings in the tab were left default. In transition settings, the "Prediction" tab was left default. In the "Filter" tab, +1, +2, +3, +5, +6 peptide precursor charges were specified. Ion charges were set to +1 and +2. Ion type was set to y and b. Product ion selection was set as follows: in the "From:" window the "ion 4" and in the "To:" window the "last ion" options were selected. The "Auto select all matching transition" option was selected at the bottom of the tab and in the "Special ions:" menu the "N-terminal to Proline" option was selected. In the "Library" tab, the "Ion match tolerance" window was set to 0.05 m/z and only peptides that have at least 4 product ions were kept in analysis. In addition, if more product ions were available per peptide, then the 6 most intense were picked up from the filtered product ions. A function of the "From filtered ion charges and types" was chosen. In the "Instrument" tab inventors included product ions from m/z 350 up to m/z 1100. The "Method match tolerance m/z" window was set to 0.055 m/z mass tolerance. MS1 filtering was set to none in the full scan tab and in MS/MS filtering section, the "Acquisition method" window the "DIA" option was selected and in the "Product mass analyzer," the "Orbitrap" option was selected. In the "Isolation scheme:" the "Add" option was selected and in the "Edit isolation scheme" pop-up window an option of the "Prespecified isolation windows" was selected. The "Import" isolation windows from DIA raw file option was then selected and the isolation scheme was read from a DIA raw file. Back in the "Full-Scan" tab in the "Resolving power:" section the resolving power was set to 30000 at 200 m/z. In the retention time filtering section, "Use only scans within 5 minutes of MS/MS IDs" was specified. Under the "Refine" tab, the "Document" section of the "Advanced" window the "Min transitions per precursor" option was set to 4. Empty proteins were removed from the document. An equal number of reverse sequence decoys via the "Add Decoys" function in the "Refine" tab was added. In the "Add Decoy Peptides" pop-up window a reverse sequence decoy generation method to create decoy peptides was selected. Following, DIA raw files were imported. mProphet model to reintegrate peptide boundaries was trained as follows: in the "Refine" tab the "Reintegrate" function was selected. In the "Reintegrate" pop-up window in the "Peak scoring model:" the "Add" option was selected. Following, in the "Edit Peak Scoring Model," mProphet model was activated. mProphet model was trained using targets and decoys. The "Score rows" with negative "Weight" and/or "Percentage Contribution" (red highlighted) were removed, and the mProphet model was then re-trained. New mProphet peak scoring model was then selected in the "Edit peak scoring Model" window and applied for reintegration of new peak boundaries. Export report function to export a summary of all dependencies required for MSstats 4.0.1. was used to generate quantitative report for downstream analysis.

### Data-Independent Acquisition (DIA) statistical data analysis in MSstats

Statistical analysis of Skyline extracted DIA data was performed in R (version 4.1.2) package MSstats 4.0.1. The protein column was combined with the peptide sequence column to preserve analysis at peptide level, this prevents summing peptide intensities originating from one protein. Extracted peakgroups were reduced by filtering on mProphet q-value 0.01 cut-off. SkylinetoMSstatsFormat function was set to keep proteins with one feature and to transform Skyline output to MSstats input. Further, peptide intensities were log2 transformed and quantile normalized. Differential immunopeptide quantitation across specified conditions was performed pairwise via mixed-effect models implemented in the MSstats "groupComparison" function. p-values were adjusted using the Benjamini-Hochberg method and output result matrix was exported for downstream analyses. Volcano plots were generated in EnhancedVolcano 1.10.0, venn diagrams in eulerr 6.1.1. package, bar graphs in plyr 1.8.6. and ggplot2 3.3.5. all running under R (version 4.1.2).

### Tissue MHC class I immunopeptidome predictions

To predict the binding affinities of peptide sequences to a set of alleles with a frequency not lower than 6.4%, inventors used the MHCflurry package (version 3.1.0) in Python 3.9.13. This package utilizes a pre-trained deep learning model and a large-scale dataset of peptide-MHC binding affinities. Peptides with predicted affinity below 50 were classified as strong binders, peptides with affinity between 50 and 150 as mid-binders, peptides with affinity between 150 and 500 as weak binders, and peptides with affinity above 500 as non-binders.

The allele-independent MHCflurry predictions utilized deep learning architectures such as convolutional neural networks (CNNs) or recurrent neural networks (RNNs) to learn complex patterns from peptide sequences and their physicochemical properties. These models were trained on diverse datasets that included peptide-MHC binding affinity measurements for different MHC alleles. By extracting relevant features from the input peptide sequences, the models made accurate predictions of peptide-MHC binding affinities without relying on specific alleles. For the presentation predictions, peptides with a allele independent presentation score above 0.8 were considered probably presented, while peptides with a allele independent presentation score below 0.8 were considered less probably presented and, therefore, HLA non-binders. The percentile distribution of MHCflurry presentation scores assessed the relative ranking of peptide presentation predictions within a diverse population of peptides in MHCflurry. Factors such as peptide sequence, length, and potential MHC binding affinity were taken into account. Peptides with a presentation percentile below 1% were considered favorable presentation percentile immunopeptides, while those above 1% were considered peptides with a non-favorable presentation percentile. Additionally, inventors employed a deep learning architecture trained on a real datasets to predict proteasomal processing. Peptides with a presentation score above 0.8 were considered probably processed, while peptides with a score below 0.8 were considered less probably processed. The results from MHCflurry were visualized using the ggplot2 R package as bar plots.

To assess MHC Class I peptide antigenicity, inventors used the IEDB MHC class I peptide antigenicity prediction in Python 3.9.13. The prediction script was obtained from the Immune Epitope Database (IEDB) website at https://www.iedb.org/. The IEDB employed computational algorithms and predictive models that considered factors such as peptide length, amino acid sequence, and MHC molecule mask to estimate peptide immunogenicity. The threshold for determining immunogenic and non-immunogenic immunopeptides was set according to recommendations in the IEDB database. Peptides with an immunogenicity score below 0 were considered non-immunogenic, while peptides with a score above 0 were considered immunogenic. The results from IEDB MHC class I peptide antigenicity predictions was visualized using the ggplot2 R package as bar plots.

The invention method of tissue MHC class I immunopeptidome by antibody free approach highlights a quantitative evaluation of complex peptide mixture to reveal the diverse nature of tissue MHC class I immunopeptidome. The invention workflow comprehensively identifies at least 15671 MHC class I peptides (with iPROB < 0.99) from 2290 protein precursors in both NSCLC tissues (n=5) and healthy donors (n=5) samples. Among these, at least 8565 MHC class I peptides were quantitated (with mPROPHET qvalue < 0.01) with comparing the healthy tissue (n=5) to the NSCLC tissue (n=5) samples. Detailed insight into the tissue MHC class I immunopeptidome peptide identification by antibody free approach shown in the figure 8. Further details and visual representations can be found in figure panel 8 A-C, which presents information on MHC class I peptide identification in individual tissue samples, the overlap of peptide identification between the NSCLC tissue and control tissue sample groups. Venn diagram highlights a considerably higher number of peptides in the NSCLC tissue group compared to healthy tissue (control tissue). Each search engine such as COMET (CMT), and MS FRAGGER (FRG), has inbuild algorithm therefore, to consider the comprehensiveness of data analysis. The invention employed two types of mass spectrometry data acquisition, data-dependent acquisition (DDA) and data-independent acquisition (DIA), then multi-search engine strategies for creating the spectral library for qualitative and quantitative tissue MHC class I immunopeptidome, facilitating discrimination between the NSCLC tissue and control tissue sample groups conditions. As a result, for the first time, comprehensive qualitative tissue MHC class I immunopeptidome are identified (figure 8). Next step is to quality control of MHC class I immunopeptidome, the peptide length distribution in between 7-16 mer amino acids is the key feature of MHC class I peptides. In the figure 9, the distribution of peptide lengths for all tissue MHC class I immunopeptidomics identified peptides is shown. The most of the peptides fall into the range of 7-16 -mers and higher the enrichment of 9-mer peptides, which is consistent with the characteristic length of MHC class I immunopeptidomes.

The tissue MHC class I immunopeptidome predictions have several platforms available to date. The invention, the MHCflurry platform was implemented to provide an overview of tissue MHC class I immunopeptide enrichment among contaminant peptides in the quantitative immunopeptidomics analysis comparing non-small cell lung cancer (NSCLC) tissue to healthy tissue (figure 10). MHCflurry utilized deep learning architectures, such as convolutional neural networks (CNNs) and recurrent neural networks (RNNs), to predict peptide-MHC binding affinities in an allele-independent manner. Additionally, ensemble models were used to capture allele-specific binding preferences, considering the unique characteristics and preferences of each MHC allele, including peptide anchor positions and residue preferences (figure 10). Antigen processing predictions were also based on machine learning models, which were trained on experimentally observed proteasome cleavage sites (figure 10). These models take into account sequence motifs, amino acid properties, and context-specific information to identify likely proteasomal cleavage sites. Furthermore, the predictions integrate the preferences of the Transporter associated with antigen processing (TAP) complex, which plays a crucial role in peptide transport for MHC loading. Moreover, the IEDB MHC class I Peptide Binding Prediction Workflow was implemented to estimate the immunogenicity of isolated peptides (figure 10). Immunogenicity determination relied on parameters such as peptide length, amino acid sequence, and MHC molecule characteristics. By considering these approaches, an insight on the extent of enrichment of immunopeptides in tissue class I immunopeptidomics samples isolated NSCLC tissue and healthy control tissue was retrieved. By integrating these predictions figure 10 A-F inventors provided a comprehensive understanding of the MHC class I immunopeptide composition of tissue immunopeptidomics isolate by antibody free approach and further offering insights into the dynamics of immune recognition and peptide-MHC class I interactions in NSCLC tissue samples.

The invention approach of quantitative tissue MHC class I immunopeptidome by antibody free approach provides a insight into neoantigen discovery. After performing a multi-bioinformatics analysis, the invention quantitative tissue MHC class I immunopeptidome workflow reveals perturbation in immunopeptidome signaling within the NSCLC tissue samples in comparison to healthy samples group, which indicating attenuation of lymphocytes-related immunopeptidome in the NSCLC tissue samples, which might be the source of neoantigen that NSCLC tumor-specific mutations or aberrantly expressed proteins, making them attractive targets for cancer immunotherapy. However, further validation are necessary to determine the immunotherapy effectiveness. In particular, the quantitative tissue MHC class I immunopeptidomics analysis aimed to identify and characterize differences in the peptide repertoire between healthy tissue and Non-Small Cell Lung Cancer (NSCLC) tissue samples. Through comprehensive analysis using Gene Set Enrichment Analysis (GSEA), a subset of significantly changed peptides and their corresponding protein precursors belonging to the lymphocyte activation term (GO:0046649) was revealed as shown in GSEA result in figure 11. The enrichment pattern suggests a decreased activity of lymphocyte-related processes in NSCLC tissue expressed by an altered immunopeptidome profile (figure 11). Moreover, figure 12 summarizes the human leukocyte antigen (HLA) system (the major histocompatibility complex [MHC] in humans) HLA binding evaluation of a subset of significantly changed MHC class I peptides associated with the lymphocyte activation term from figure 11. The figure 12, demonstrates the differential regulation of HLA binders originating from this term in healthy tissue compared to NSCLC tissue samples. Notably, HLA binders associated with the lymphocyte activation term were more frequently upregulated in healthy tissue, indicating an increased MHC class I immunopeptide presentation. In contrast, a population of MHC class I peptides with opposing regulation was observed, but in general most of these peptides were predicted not to bind to HLA molecules. This suggests that this population of peptides contains a higher proportion of contaminants and may not be directly involved in the changed presentation patterns representing lymphocyte deactivation in NSCLC samples. The differential regulation observed in HLA binders associated with the lymphocyte activation term further supports the hypothesis of lymphocyte attenuation in NSCLC tissue samples in comparison with healthy tissue samples and further, the inventors provide a list of immunopeptide biomarkers/neoantigens (Table 5).

Lastly, the inventors selected several unique MHC class I peptidome as the source of possible neoantigen peptidome and biomarker candidates for NSCLC therapy or diagnosis, respectively. Figure 13 presents a volcano plot depicting the dysregulated protein sources and their corresponding MHC class I immunopeptides sequences derived from protein precursors of the lymphocyte activation term (GO:0046649) for diagnosing lymphocyte attenuation in Non-Small Cell Lung Cancer (NSCLC), which distinguishing NSCLC tissue from healthy tissue (figure 11, 12) samples. The volcano plot specifically focuses on selected targets that are proposed in inventions, representing immunopeptides originating from protein precursors linked by the lymphocyte activation term (GO:0046649). Figure 13, the volcano plot shows the fold change in expression or regulation (x-axis) versus the statistical significance (y-axis) of the dysregulated MHC class I immunopeptides. Each point on the plot represents an immunopeptide, with its position indicating both the magnitude of regulation and the level of statistical significance. The selected labels on the plot highlight the regulation of MHC class I immunopeptides derived from protein precursors associated with the lymphocyte activation term (GO:0046649). These labels provide important information about the upregulation and downregulation of specific MHC class I immunopeptides and their potential relevance in the context of lymphocyte activation and NSCLC samples. Therefore, all these at least 25 quantitative immunopeptidome (Table 5), HLA binder, and the peptide length between 7-16 amino acids (majority at 9 mers) MHC class I immunopeptidome patterns for lymphocyte-related immune responses (lymphocyte attenuation) in NSCLC.

**Table 5. MHC class I quantitative tissue immunopeptides therapeutic panel for NSCLC. The sequence of therapeutic MHC class I peptides, source protein identifier for each peptide, length, as well as HLA binding prediction evaluation and fold changes of peptides either up-regulated (T), down-regulated (↓) in comparison of healthy tissue to non-small cell lung cancer (NSCLC) tissue samples. The regulation/fold change refers only to MHC class I tissue quantitative peptides. It shows how many times more signaling intensities present in the tissue of healthy samples were higher than in non-small cell lung cancer (NSCLC) tissue samples. These at least 25 quantitative immunopeptidome presented in table 5 disclosing MHC class I immunopeptide sequences derived from protein precursors of the lymphocyte activation term for diagnosing lymphocyte attenuation in Non-Small Cell Lung Cancer (NSCLC) tissue samples.**

| Source protein | Peptide | Length | HLA binding evaluation | Regulation (control vs. NSCLC tumor) |
|---|---|---|---|---|
| MYH9_HUMAN | Lys-Pro-Ala-Gly-Pro-Pro-Gly-Ile-Leu-Ala-Leu | 11 | Binder | ↑ |
| LEG1_HUMAN | Tyr-Glu-Phe-Lys-Phe-Pro-Asn-Arg-Leu | 9 | Binder | ↑ |
| SPT6H_HUMAN | Asp-Val-Tyr-Asn-His-Phe-Leu-Leu-Tyr | 9 | Binder | ↑ |
| RAGE_HUMAN | Phe-Thr-Leu-Gln-Ser-Glu-Leu | 7 | Binder | ↑ |
| TYY1_HUMAN | Lys-Glu-Asp-Asp-Ala-Pro-Arg-Thr-Ile | 9 | Binder | ↑ |
| B2MG_HUMAN | Thr-Glu-Phe-Thr-Pro-Thr-Glu-Lys | 8 | Binder | ↑ |
| ITB1_HUMAN | Phe-Arg-Ile-Gly-Phe-Gly-Ser-Phe | 8 | Binder | ↑ |
| KSYK_HUMAN | Lys-Thr-Asn-Gly-Lys-Phe-Leu-Ile-Arg | 9 | Binder | ↑ |
| BAG6_HUMAN | Met-Pro-Val-Gly-Pro-Asp-Ala-Ile-Leu-Arg-Tyr | 11 | Binder | ↑ |
| MYH9_HUMAN | Gln-Gly-Phe-Pro-Asn-Arg-Val-Val-Phe | 9 | Binder | ↑ |
| CASL_HUMAN | Tyr-Val-Tyr-Glu-Tyr-Pro-Ser-Arg-Tyr | 9 | Binder | ↑ |
| DRA_HUMAN | Asn-Ala-Ala-Glu-Arg-Arg-Gly-Pro-Leu | 9 | Binder | ↑ |
| DPB1_HUMAN | Ala-Pro-Arg-Thr-Val-Ala-Leu-Thr-Ala | 9 | Binder | ↑ |
| BAG6_HUMAN | Met-Pro-Val-Gly-Pro-Asp-Ala-Ile-Leu-Arg-Tyr | 11 | Binder | ↑ |
| ANXA1_HUMAN | Thr-Ile-Ile-Asp-Ile-Leu-Thr-Lys-Arg | 9 | Binder | ↑ |
| STAT3_HUMAN | Arg-Tyr-Leu-Glu-Lys-Pro-Met-Glu-Ile | 9 | Binder | ↑ |
| DOA_HUMAN | Gly-Thr-Tyr-Val-Ser-SerVal-Pro-Arg | 9 | Binder | ↑ |
| FYN_HUMAN | Gly-Thr-Asp-Pro-Thr-Pro-Gln-Hi s-Tyr | 9 | Binder | ↑ |
| ANXA1_HUMAN | Ala-Ile-Leu-Asp-Glu-Thr-Lys-Gly-Asp-Tyr | 10 | Binder | ↓ |
| GELS_HUMAN | Val-Leu-Lys-Thr-Pro-Ser-Ala-Ala-Tyr | 9 | Binder | ↓ |
| GELS_HUMAN | Asp-Leu-Val -Pro-Val -Pro-Thr-Asn-Leu | 9 | Binder | ↓ |
| GELS_HUMAN | Val-Ile-Glu-Glu-Val-Pro-Gly-Glu-Leu | 9 | Binder | ↓ |
| ANXA1_HUMAN | Tyr-Gln-Lys-Met-Tyr-Gly-Ile-Ser-Leu | 9 | Binder | ↓ |
| SODC_HUMAN | Lys-Gly-Asp-Gly-Pro-Val-Gln-Gly-Ile-Ile-Asn-Phe | 12 | Binder | ↓ |
| RS6_HUMAN | Ile-Glu-Val-Asp-Asp-Glu-Arg-Lys-Leu-Arg-Thr-Phe | 12 | Binder | ↓ |

The finding of potential mechanisms underlying lymphocyte dysfunction in NSCLC offers the implication of these immunopeptides for health status evaluation, disease diagnostics, neoantigen discovery and therapeutic strategies to address lymphocyte-related immune responses in NSCLC.

### References

[1] R. Richter et al., "Composition of the peptide fraction in human blood plasma: database of circulating human peptides," J. Chromatogr. B. Biomed. Sci. App., vol. 726, no. 1, pp. 25-35, Apr. 1999, doi: 10.1016/S0378-4347(99)00012-2.
[2] T. Cardon et al., "Optimized Sample Preparation Workflow for Improved Identification of Ghost Proteins," Anal. Chem., 2020, doi: 10.1021/acs.analchem.9b04188.
[3] B. Fabre, J.-P. Combier, and S. Plaza, "Recent advances in mass spectrometry-based peptidomics workflows to identify short-open-reading-frame-encoded peptides and explore their functions," Curr. Opin. Chem. Biol., vol. 60, pp. 122-130, Feb. 2021, doi: 10.1016/j.cbpa.2020.12.002.
[4] A. W. Purcell, S. H. Ramarathinam, and N. Ternette, "Mass spectrometry-based identification of MHC-bound peptides for immunopeptidomics," Nat. Protoc., vol. 14, no. 6, pp. 1687-1707, Jun. 2019, doi: 10.1038/s41596-019-0133-y.
[5] F.-R. Schumacher et al., "Building proteomic tool boxes to monitor MHC class I and class II peptides," Proteomics, vol. 17, no. 1-2, Jan. 2017, doi: 10.1002/pmic.201600061.
[6] M. Bassani-Sternberg, S. Pletscher-Frankild, L. J. Jensen, and M. Mann, "Mass spectrometry of human leukocyte antigen class I peptidomes reveals strong effects of protein abundance and turnover on antigen presentation," Mol. Cell. Proteomics MCP, vol. 14, no. 3, pp. 658-673, Mar. 2015, doi: 10.1074/mcp.M114.042812.
[7] D. Gebreselassie, H. Spiegel, and S. Vukmanovic, "Sampling of major histocompatibility complex class I-associated peptidome suggests relatively looser global association of HLA-B∗5101 with peptides," Hum. Immunol., vol. 67, no. 11, pp. 894-906, Nov. 2006, doi: 10.1016/j.humimm.2006.08.294.
[8] L. Wu et al., "Peptidomic Analysis of Cultured Cardiomyocytes Exposed to Acute Ischemic-Hypoxia," Cell. Physiol. Biochem., vol. 41, no. 1, pp. 358-368, 2017, doi: 10.1159/000456282.
[9] Y. Xue et al., "Peptidomic Analysis of Endometrial Tissue from Patients with Ovarian Endometriosis," Cell. Physiol. Biochem. Int. J. Exp. Cell. Physiol. Biochem. Pharmacol., vol. 47, no. 1, pp. 107-118, 2018, doi: 10.1159/000489753.
[10] G. P. Roberts et al., "Comparison of Human and Murine Enteroendocrine Cells by Transcriptomic and Peptidomic Profiling," Diabetes, vol. 68, no. 5, Art. no. 5, May 2019, doi: 10.2337/db18-0883.
[11] C.-X. Zhou, M. Gao, B. Han, H. Cong, X.-Q. Zhu, and H.-Y. Zhou, "Quantitative Peptidomics of Mouse Brain After Infection With Cyst-Forming Toxoplasma gondii," Front. Immunol., vol. 12, 2021, Accessed: Feb. 15, 2023. [Online]. Available: https://www.frontiersin.org/articles/10.3389/fimmu.2021.681242
[12] L. Zhang et al., "Peptidomics Analysis Reveals Peptide PDCryab1 Inhibits Doxorubicin-Induced Cardiotoxicity," Oxid. Med. Cell. Longev., vol. 2020, p. 7182428, Oct. 2020, doi: 10.1155/2020/7182428.
[13] Y. Xue et al., "Peptidomic Analysis of Endometrial Tissue from Patients with Ovarian Endometriosis," Cell. Physiol. Biochem., vol. 47, no. 1, pp. 107-118, 2018, doi: 10.1159/000489753.
[14] J. Hu et al., "Peptidomic analysis on synovial tissue reveals galectin-1 derived peptide as a potential bioactive molecule against rheumatoid arthritis," Cytokine, vol. 131, p. 155020, Jul. 2020, doi: 10.1016/j.cyto.2020.155020.
[15] F. Zhang et al., "Peptidome Analysis of Pancreatic Tissue Derived from T1DM Mice: Insights into the Pathogenesis and Clinical Treatments of T1DM," BioMed Res. Int., vol. 2021, p. 9987042, May 2021, doi: 10.1155/2021/9987042.
[16] X. Hua et al., "Peptidome analysis of human intrauterine adhesion tissues and the identification of antifibrotic peptide," J. Biomed. Res., vol. 36, no. 4, pp. 280-296, Jul. 2022, doi: 10.7555/JBR.36.20220059.
[17] T. Chen, Z. Zhang, Q. Lu, and J. Ma, "Screening and functional analysis of the differential peptides from the placenta of patients with healthy pregnancy and preeclampsia using placental peptidome," Front. Genet., vol. 13, p. 1014836, 2022, doi: 10.3389/fgene.2022.1014836.
[18] X. Li et al., "Comparative peptidome profiling reveals critical roles for peptides in the pathology of pancreatic cancer," Int. J. Biochem. Cell Biol., vol. 120, p. 105687, Mar. 2020, doi: 10.1016j.biocel.2020.105687.
[19] S. Dasgupta et al., "Proteasome Inhibitors Alter Levels of Intracellular Peptides in HEK293T and SH-SY5Y Cells," PLOS ONE, vol. 9, no. 7, p. e103604, Jul. 2014, doi: 10.1371/journal.pone.0103604.
[20] T. GARCÍA-BERROCOSO and J. M. Vilallonga, "Biomarkers for the prognosis of ischemic stroke," WO2014064202A1, May 01, 2014 Accessed: Feb. 15, 2023. [Online]. Available: https://patents.google.com/patent/WO2014064202A1/en?oq=WO2014%2f064202A1
[21] C. Watson, M. Ledwidge, K. McDonald, and J. Baugh, "Biomarkers of cardiovascular disease including lrg," US20130084276A1, Apr. 04, 2013 Accessed: Feb. 15, 2023. [Online]. Available: https://patents.google.com/patent/US20130084276A1/en?oq=US2013%2f0084276A1

## Claims

1. Method for screening of extracellular tissue peptides in mammalian tissue samples for health status evaluation, disease diagnostics and neoantigen discovery by amino acid sequencing with tandem mass spectrometry with signaling intensities measurement of peptides **characterized in that** the two steps of sample preparation are performed as follows:
a) the tissue samples are subjected to 2-3 washes with cleaned phosphate-buffered saline (PBS) pH 7.2-7.5.
and tissue samples are incubated with an extraction buffer in the form of citrate-phosphate buffer with pH 3.1-3.6 prepared in ultrapure water, purity >_ 99.9%, wherein the buffer is at a volume of 7-8 ml to 0.05-1 gram of tissue sample and incubation step is performed by 2-3 minutes with mixing at 4 to 10 °C;
b) purification of isolated tissue extracellular peptidome is performed by using hydrophilic-lipophilic balanced columns for 120-180 minutes at room temperature and further separated with ultrafiltration using selected molecular weight cutoff filter that has 3kDa for 120-135 minutes centrifuge at least 4000 g, at 4 - 10 °C.

2. Method according to claim 1 , wherein
a) performing extraction in a way that the tissue samples are subjected to 7-10 washes with phosphate-buffered saline (PBS) without additives (no calcium/magnesium/phenol red) pH 7.2-7.5, then
tissue samples are incubated with an extraction buffer (citrate-phosphate buffer) with pH 3.3 prepared in liquid chromatography-mass spectrometry (LC-MS) grade ultrapure water, purity >_ 99.9%, wherein the buffer is at a volume of 5-6 ml to 0.5-1 gram of tissue sample and incubation step takes 4-5 minutes with mixing at a temperature of 4 to 10 ° C,
while purification of isolated tissue MHC class I immunopeptidome is performed by using hydrophilic-lipophilic balanced columns for 120-180 minutes at room temperature and further separated with ultrafiltration using selected molecular weight cutoff filter that has 3kDa molecular weight filters to for 120-135 minutes centrifuge at least 4000 g, at 4 - 10 °C.
